(19) Europäisches Patentamt
European Patent Office
Office européen des brevets

(11) EP 4 204 407 B1

(12) **EUROPEAN PATENT SPECIFICATION**

(45) Date of publication and mention of the grant of the patent:
**19.06.2024 Bulletin 2024/25**

(21) Application number: **21769101.3**

(22) Date of filing: **24.08.2021**

(51) International Patent Classification (IPC):
***C07D 401/04*** *(2006.01)*

(52) Cooperative Patent Classification (CPC):
**C07D 401/04**

(86) International application number:
**PCT/EP2021/073378**

(87) International publication number:
**WO 2022/043320 (03.03.2022 Gazette 2022/09)**

(54) **PROCESS**

VERFAHREN

PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.08.2020 GB 202013383**

(43) Date of publication of application:
**05.07.2023 Bulletin 2023/27**

(73) Proprietor: **Givaudan SA**
**1214 Vernier (CH)**

(72) Inventors:
- **LOVCHIK, Martin**
  **8600 Duebendorf (CH)**
- **GRANIER, Thierry**
  **8600 Duebendorf (CH)**
- **JOSET, Nathalie**
  **8600 Duebendorf (CH)**

(74) Representative: **Global Patents**
**Givaudan SA**
**Grafenaustrasse 7**
**6300 Zug (CH)**

(56) References cited:
**WO-A1-2013/163270 WO-A1-2015/047973**
**WO-A1-2021/074281**

- **JOHN O LINK ET AL: "Discovery of Ledipasvir (GS-5885): A Potent, Once-Daily Oral NS5A Inhibitor for the Treatment of Hepatitis C Virus Infection", JOURNAL OF MEDICINAL CHEMISTRY, AMERICAN CHEMICAL SOCIETY, US, vol. 57, no. 5, 13 March 2014 (2014-03-13) , pages 2033-2046, XP002756084, ISSN: 0022-2623, DOI: 10.1021/JM401499G [retrieved on 2014-01-10]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 204 407 B1

## Description

### Technical Field

The present invention relates generally to a method for the *in-situ* formation of compounds of formula (V) as described herein from pipecolic acid, particularly the formation of the cooling agents 2-methyl-1-(2-(5-(p-tolyl)-1H-imidazol-2-yl)piperidin-1-yl)butan-1-one (including (2S)-2-methyl-1-(2-(5-(p-tolyl)-1H-imidazol-2-yl)piperidin-1-yl)butan-1-one, or a racemic mixture) , 2-(methylthio)-1-(2-(5-(p-tolyl)-1H-imidazol-2-yl)piperidin-1-yl)propan-1-one, 2-methyl-2-(methylthio)-1-(2-(5-(p-tolyl)-1H-imidazol-2-yl)piperidin-1-yl)propan-1-one and, 2,2-dimethyl-1-(2-(5-(p-tolyl)-1H-imidazol-2-yl)piperidin-1-yl)but-3-en-1-one. The present invention further relates to the compounds obtained by and/or obtainable by said methods, the use of said compounds in flavor compositions, for example as cooling agents in flavor compositions, the use of said flavor compositions in consumer products, flavor compositions comprising said compounds, and consumer products comprising said flavor compositions.

### BACKGROUND

Pipecolic acid may be used as a starting material for the formation of various compounds. In particular, pipecolic acid may be used as a starting material for the formation of compounds of formula (V) as described herein. However, these methods generally require a number of steps involving the isolation and purification of the intermediate compounds before the next step can be performed. In this respect reference is made to WO2015/047973. It is therefore desirable to provide improved or alternative methods, which may, for example, reduce the need to isolate and purify the intermediate compounds.

### SUMMARY

In accordance with a first aspect of the present invention there is provided a method for the *in-situ* formation of a compound of formula (V) from pipecolic acid, wherein the *in-situ* method takes place in the presence of a solvent, wherein the solvent is an organic solvent having a boiling point ranging from about 50°C to about 160°C, water, or a mixture thereof, wherein the method comprises:

(a) reacting pipecolic acid with an acid chloride of formula (Ia) in the presence of a base, or reacting pipecolic acid with an acid anhydride of formula (Ib), optionally in the presence of a base, to form a compound of formula (II),
(b) reacting the compound of formula (II) with a compound of formula (III) to form a compound of formula (IV), and
(c) reacting the compound of formula (IV) with an ammonium source to form a compound of formula (V),

wherein the structures of the compounds of formula (Ia), (Ib), (II), (III), (IV) and (V) are as follows:

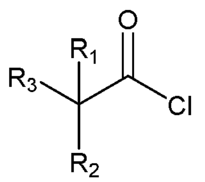

Formula (Ia)

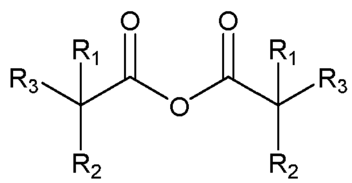

Formula (Ib)

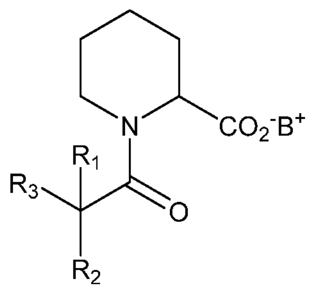

Formula (II)

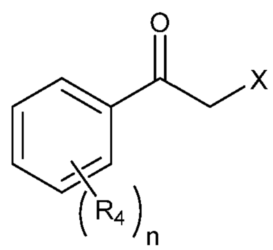

Formula (III)

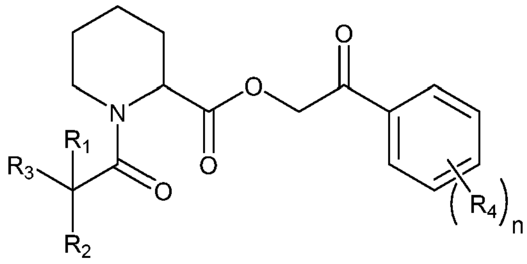

Formula (IV)

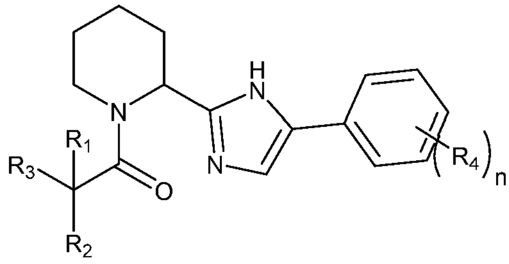

Formula (V)

wherein

$R_1$, $R_2$ and $R_3$ together with the carbon atom to which they are attached form a hydrocarbon group optionally comprising up to 3 heteroatoms independently selected from O, S, N and F; the phenyl group of the compounds of formulas (III), (IV) and (V) is substituted with n $R_4$ substituents wherein n is zero, one, two, three, four or five;
each $R_4$ is independently selected from halogen, cyano, nitro, $C_1$-$C_6$ alkyl optionally comprising up to 5 halogen atoms, $C_2$-$C_6$ alkenyl, $C_1$-$C_6$ alkoxy optionally comprising up to 3 halogen atoms, $C_1$-$C_3$-alkoxy-$C_1$-$C_3$-alkyl, and $C_3$-$C_7$ cycloalkyl;
$B^+$ is a cation provided by the base; and
X is a halogen.

In accordance with a second aspect of the present invention there is provided a compound of formula (V) obtained by and/or obtainable by the method of the first aspect of the present invention, including any embodiment thereof.

In accordance with a third aspect of the present invention there is provided the use of a compound of formula (V) of the second aspect of the present invention, including any embodiment thereof, in a flavor composition. For example, the compound of formula (V) may be used as a cooling agent in a flavor composition.

In accordance with a fourth aspect of the present invention there is provided a flavor composition comprising a compound of formula (V) of the second aspect of the present invention, including any embodiment thereof.

In accordance with a fifth aspect of the present invention there is provided a consumer product comprising a flavor composition of the fourth aspect of the present invention, including any embodiment thereof.

In accordance with a sixth aspect of the present invention there is provided a use of a flavor composition of the fourth aspect of the present invention, including any embodiment thereof, in a consumer product.

Certain embodiments of the present invention may provide one or more of the following advantages:

- *in-situ* method for making a compound of formula (V) from pipecolic acid;
- reduction in the number of purification and isolation steps;
- use of the same solvent throughout the method;
- solvent can be recycled at the end of the reaction;
- the chirality of the hydrocarbon group formed by $R_1$, $R_2$ and $R_3$ stays the same throughout the reaction;
- the acid chloride and compounds of formula (III) can be made in-house and can be used crude and in solution, thus reducing exposure to lachrymal reagents;
- reduced hydrolysis of the acid chloride as a side reaction;
- milder reaction conditions;
- reduced amount or number of reagents required (e.g. the carboxylic acid is deprotonated in the first reaction step so there is no need to add additional base in the second reaction step);
- reduced amount of ammonium source required for the third reaction step (e.g. due to the addition in portions and/or the reaction temperature and/or the removal of water);
- acceptable yield;
- acceptable selectivity.

The details, examples and preferences provided in relation to any particular one or more of the stated aspects of the present invention will be further described herein and apply equally to all aspects of the present invention. Any combination of the embodiments, examples and preferences described herein in all possible variations thereof is encompassed by the present invention unless otherwise indicated herein, or otherwise clearly contradicted by context.

## DETAILED DESCRIPTION

The present invention is based on the surprising finding that compounds of formula (V) can be formed *in-situ* from pipecolic acid prior to work-up and isolation.

There is therefore provided herein a method for the *in-situ* formation of a compound of formula (V) from pipecolic acid, wherein the *in-situ* method takes place in the presence of a solvent, wherein the solvent is an organic solvent having a boiling point ranging from about 50°C to about 160°C, water, or a mixture thereof, wherein the method comprises:

(a) reacting pipecolic acid with an acid chloride of formula (Ia) in the presence of a base, or reacting pipecolic acid with an acid anhydride of formula (Ib), optionally in the presence of a base, to form a compound of formula (II),

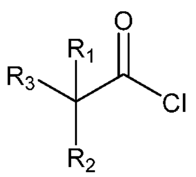
Formula (Ia)

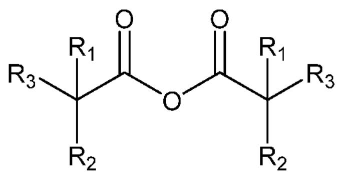
Formula (Ib)

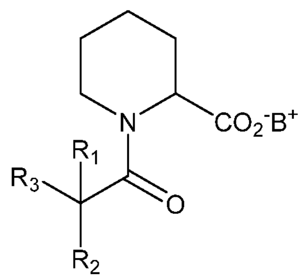
Formula (II)

(b) reacting the compound of formula (II) with a compound of formula (III) to form a compound of formula (IV),

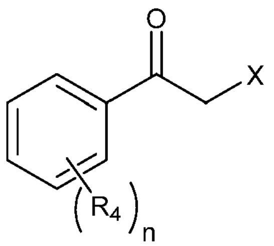
Formula (III)

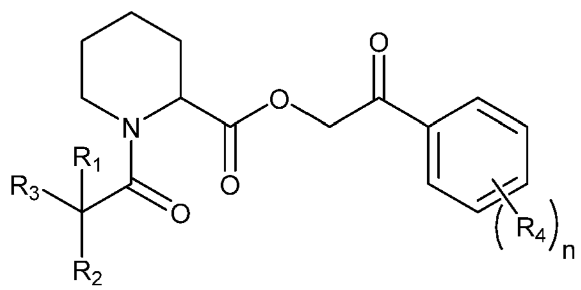
Formula (IV)

(c) reacting the compound of formula (IV) with an ammonium source to form a compound of formula (V),

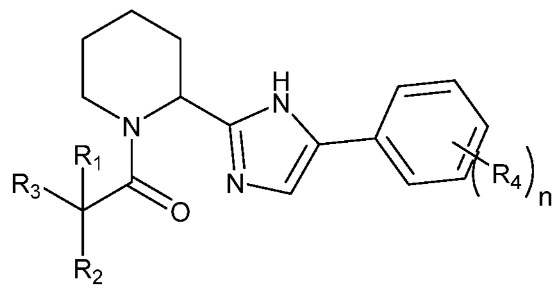
Formula (V)

wherein

$R_1$, $R_2$ and $R_3$ together with the carbon atom to which they are attached form a hydrocarbon group optionally comprising up to 3 heteroatoms independently selected from O, S, N and F; the phenyl group of the compounds of formulas (III), (IV) and (V) is substituted with n $R_4$ substituents wherein n is zero, one, two, three, four, or five; each $R_4$ is independently selected from halogen, cyano, nitro, $C_1$-$C_6$ alkyl optionally comprising up to 5 halogen atoms, $C_2$-$C_6$ alkenyl, $C_1$-$C_6$ alkoxy optionally comprising up to 3 halogen atoms, $C_1$-$C_3$-alkoxy-$C_1$-$C_3$-alkyl,

and $C_3$-$C_7$ cycloalkyl;
$B^+$ is a cation provided by the base; and
X is a halogen.

As used herein the phrase "*in-situ* formation of a compound of formula (V) from pipecolic acid" refers to a method wherein the entire reaction takes place in a single reaction mixture and any intermediate compounds that are formed (*e.g*. compounds of formula (II), and (IV)) are not isolated or purified before the subsequent steps are carried out to form the final product (*i.e*. the compound of formula (V)). In other words, the conversion of the pipecolic acid to the compound of formula (II), the conversion of the compound of formula (II) to the compound of formula (IV), and the conversion of the compound of formula (IV) to the compound of formula (V) takes place in the same reaction mixture without isolation or purification of the compound of formula (II) or the compound of formula (IV). This in-situ formation may take place, for example, in flow or a batch process.

Although the methods described herein are *in-situ* methods for the formation of a compound of formula (V) from pipecolic acid, it will be recognised that this is not necessary to obtain the compound of formula (V) and one or more isolation or purification steps could be performed after each of steps 1, 2 and 3 described herein in order to obtain a compound of formula (V).

The conversion of the compound of formula (II) to the compound of formula (IV) may be concomitant with or subsequent to the conversion of the pipecolic acid to the compound of formula (II).

The conversion of the compound of formula (IV) to the compound of formula (V) may be concomitant with or subsequent to the conversion of the compound of formula (II) to the compound of formula (IV).

By "concomitant with" it is meant that the reagents for each conversion step are added to the reaction mixture at the same time such that both conversion reactions take place in the reaction mixture at the same time.

By "subsequent to" it is meant that at least some of the reagents (e.g. the acid chloride of formula (Ia), the acid anhydride of formula (Ib), the compound of formula (III) and/or the ammonium source) for the next step of the reaction are added to the reaction mixture after the first step of the reaction has been partially or fully completed. For example, the compound of formula (III) may be added to the reaction mixture after complete or incomplete conversion of the pipecolic acid to the compound of formula (II). For example, the ammonium source may be added to the reaction mixture after complete or incomplete conversion of the compound of formula (II) to the compound of formula (IV). Where the previous conversion was incomplete, addition of the reagents for the subsequent step may result in a period in which both conversion reactions take place in the reaction mixture at the same time. However, there is first a period in which the previous conversion step takes place in the absence of the subsequent conversion step. The subsequent conversion takes place in the same reaction mixture and no isolation or purification of the intermediate compounds takes place, therefore the subsequent conversion is an *in-situ* reaction in accordance with the present disclosure.

Alternatively, the method for the *in-situ* formation of a compound of formula (V) as hereinabove described comprises:

(a) reacting pipecolic acid with a compound of formula (III) in the presence of a base to form a compound of formula (VI),

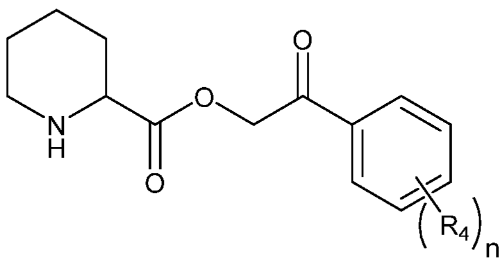

Formula (VI)

(b) reacting the compound of formula (VI) with an acid chloride of formula (Ia) or an acid anhydride of formula (Ib) to form a compound of formula (IV), and
(c) reacting the compound of formula (IV) with an ammonium source to form a compound of formula (V),

wherein the structures of the compounds of formula (Ia), (Ib), (III), (IV) and (V) are as indicated above.

In this alternative method the reaction step 3 with the ammonium source optionally could be done before the reaction with acid chloride / acid anhydride.

However, for the alternative method the pipecolic acid has to be protected by selective amine protection group.

The protecting group should then be removed prior to the subsequent reaction with an acid chloride of formula (Ia) or an acid anhydrid of formula (Ib).

The *in-situ* method described herein takes place in the presence of a solvent, wherein the solvent is an organic solvent having a boiling point ranging from about 50°C to about 160°C, or wherein the solvent is water, or wherein the solvent is a mixture of organic solvent and water. In other words, all of the steps of the method (including all of steps 1, 2 and 3 described herein) take place in the presence of a solvent, wherein the solvent is an organic solvent having a boiling point ranging from about 50°C to about 160°C, or wherein the solvent is water, or wherein the solvent is a mixture of organic solvent and water. It has surprisingly and advantageously been found that the selection of water or an organic solvent having a boiling point ranging from about 50°C to about 160°C as the solvent makes a change of solvent for each subsequent step unnecessary and thus enables the entire method to be performed in one pot as an *in-situ* method. The selection of water or an organic solvent having a boiling point ranging from about 50°C to about 160°C as the solvent may also assist in minimizing the formation of impurities and side products. In certain embodiments, the solvent is an organic solvent having a boiling point ranging from about 50°C to about 160°C.

In certain embodiments, the organic solvent has a boiling point equal to or greater than about 60°C or equal to or greater than about 70°C or equal to or greater than about 80°C or equal to or greater than about 90°C or equal to or greater than about 100°C or equal to or greater than about 110°C.

In certain embodiments, the organic solvent has a boiling point equal to or less than about 150°C or equal to or less than about 140°C or equal to or less than about 130°C or equal to or less than about 120°C.

For example, the organic solvent may have a boiling point ranging from about 60°C to about 150°C or from about 70°C to about 120°C or from about 80°C to about 140°C or from about 90°C to about 130°C or from about 100°C to about 120°C.

One or more of the steps of the *in-situ* method described herein (e.g. all of steps 1, 2 and 3 described herein or all of the steps of the method) may take place at a temperature that is less than the boiling point of the solvent, for example less than the boiling point of the organic solvent having a boiling temperature ranging from about 50°C to about 160°C. Alternatively, one or more of the steps of the *in-situ* method described herein (e.g. all of steps 1, 2 and 3 described herein or all of the steps of the method) may take place under condition below reflux. This may be to prevent the solvent from being lost from the reaction mixture during the method due to evaporation.

One or more of the steps of the *in-situ* method described herein (e.g. all of steps 1, 2 and 3 described herein or all of the steps of the method) may take place in closed tubes or in an autoclave to prevent the solvent from being lost from the reaction mixture during the reaction. This may, for example, enable temperatures higher than the boiling temperature of the solvent to be used. Therefore, solvents having relatively low boiling points (e.g. MTBE) may be used.

One or more of the steps of the *in-situ* method described herein (e.g. all of steps 1, 2 and 3 described herein or all of the steps of the method) may take place at a temperature that is less than the temperature at which the reactants decompose. The temperature of each step of the method may be the same or different.

It may, for example, be advantageous to select a solvent (e.g. an organic solvent having a boiling point ranging from about 50°C to about 160°C) which allows each step of the *in-situ* method to be carried out at a temperature that maximizes the reaction speed, but is low enough to avoid decomposition of the reactants.

The organic solvent having a boiling point ranging from about 50°C to about 160°C may, for example, be a polar or a non-polar solvent. The polarity of the solvent may be measured by determining the solvent's dielectric constant (relative permittivity) at 0°C. Solvents with a dielectric constant of less than 15 may be considered to be non-polar solvents. Solvents with a dielectric constant of 15 or more may be considered to be polar solvents.

The organic solvent having a boiling point ranging from about 50°C to about 160°C may, for example, be immiscible with water (*i.e.* it is not possible for a mixture of the organic solvent having a boiling point ranging from about 50°C to about 160°C to mix with water in all proportions to form a homogenous solution).

The organic solvent having a boiling point ranging from about 50°C to about 160°C may, for example, be an aromatic solvent or a non-aromatic solvent.

The aromatic solvent may comprise one or more heteroatoms, for example selected from nitrogen, oxygen, sulphur, and halogens such as fluorine. The aromatic solvent may, for example, comprise one or more heteroaromatic groups. The aromatic solvent may, for example, be an aromatic solvent comprised of only carbon and hydrogen atoms. Alkylbenzenes are examples of aromatic solvents comprised of only carbon and hydrogen atoms. Alkylbenzene solvents are also examples of non-polar solvents. Alkylbenzene solvents comprise a benzene group wherein one or more hydrogen atoms on the benzene ring is/are replaced with an alkyl group. Each alkyl group may, for example, independently comprise from 1 to 5 carbon atoms, for example from 1 to 3 carbon atoms, for example 1 or 2 carbon atoms. Toluene and xylene are examples of alkylbenzene solvents. Halobenzene (benzene having one or more hydrogen atoms substituted with a halogen atom) solvents such as dichlorobenzene are examples of aromatic solvents.

The non-aromatic solvent may comprise one or more heteroatoms, for example selected from nitrogen, oxygen, sulphur, and halogens such as fluorine. The non-aromatic solvent may, for example, comprise one or more non-aromatic heterocyclic groups. Methyltetrahydrofuran, for example 2-methyltetrahydrofuran, is an example of a non-aromatic solvent comprising a heterocyclic group. The non-aromatic solvent may, for example, be an ether. Methyl tert-butyl ether (MTBE) is an example of a non-aromatic ether solvent. The non-aromatic solvent may, for example, be a non-aromatic solvent comprised of only carbon and hydrogen atoms. The non-aromatic solvent comprised of only carbon and hydrogen atoms may, for example, be linear (e.g. branched or straight chain) or cyclic. Heptane is an example of a linear straight chain non-aromatic solvent. Haloalkane (alkanes having one or more hydrogen atoms substituted with a halogen atom) solvents such as dichloromethane are examples of non-aromatic solvents. Haloalkane solvents may, for example, particularly be used if the method is carried out in a closed tube or autoclave.

In certain embodiments, the non-aromatic solvent may comprise one or more heteroatoms selected from nitrogen, oxygen and sulphur.

In particular, the organic solvent having a boiling point ranging from about 50°C to about 160°C may be an alkylbenzene solvent. For example, the organic solvent having a boiling point ranging from about 50°C to about 160°C may be toluene.

*Step 1 - Conversion of pipecolic acid to a compound of formula (II)*

The *in-situ* method described herein may comprise reacting pipecolic acid with (a-i) an acid chloride of formula (Ia) in the presence of a base, or (a-ii) an acid anhydride of formula (Ib), optionally in the presence of a base, to form a compound of formula (II). For example, the *in-situ* method described herein may comprise reacting pipecolic acid with an acid chloride of formula (Ia) in the presence of a base to form a compound of formula (II).

Pipecolic acid may be obtained commercially. It has the following chemical structure:

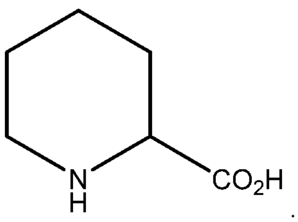

The acid chloride of formula (Ia) has the following chemical structure,

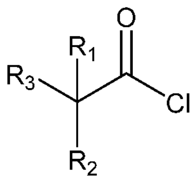

Formula (Ia)

wherein $R_1$, $R_2$ and $R_3$ together with the carbon atom to which they are attached form a hydrocarbon group optionally comprising up to 3 heteroatoms independently selected from O, S, N and F.

The acid chloride of formula (Ia) may, for example, be obtained commercially or may, for example, be made by numerous synthetic routes such as by reacting a carboxylic acid with thionyl chloride ($SOCl_2$), oxalyl chloride ($COCl_2$), phosphorus trichloride ($PCl_3$) or phosphorus pentachloride ($PCl_5$), or by reacting a thiolactic acid with dimethylsulfate followed by chlorination, for example with thionyl chloride ($SOCl_2$).

The acid anhydride of formula (Ib) has the following chemical structure,

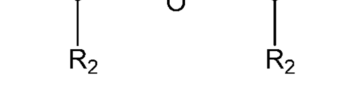

Formula (Ib)

wherein $R_1$, $R_2$ and $R_3$ are as defined in relation to the acid chloride of formula (Ia).

The acid anhydride may, for example, be obtained commercially or may, for example, be made by numerous

synthetic routes such as by reacting an acid chloride of formula (Ia) with the corresponding carboxylic acid, or the later with acetic anhydride.

The term "hydrocarbon group optionally comprising up to 3 heteroatoms selected from O, S, N and F" refers to a group comprising only carbon and hydrogen atoms and optional oxygen, sulphur, nitrogen and fluorine atoms. The maximum number of total oxygen, sulphur, nitrogen and fluorine atoms is three.

$R_1$, $R_2$ and $R_3$ together with the carbon atom to which they are attached may, for example, form a hydrocarbon group optionally comprising up to 3 heteroatoms selected from O, S and F. $R_1$, $R_2$ and $R_3$ together with the carbon atom to which they are attached may, for example, form a hydrocarbon group optionally comprising up to 3 heteroatoms which are S.

$R_1$, $R_2$ and $R_3$ together with the carbon atom to which they are attached may, for example, form a hydrocarbon group comprising zero heteroatoms (*i.e.* form a hydrocarbon group comprising only carbon and hydrogen atoms).

$R_1$, $R_2$ and $R_3$ together with the carbon atom to which they are attached may, for example, form a hydrocarbon group comprising carbon and hydrogen atoms with one or two heteroatoms independently selected from O, S, N and F. $R_1$, $R_2$ and $R_3$ together with the carbon atom to which they are attached may, for example, form a hydrocarbon group comprising carbon and hydrogen atoms with one or two heteroatoms independently selected from O, S and F.

The carbon atom to which $R_1$, $R_2$ and $R_3$ are attached may, for example, be a chiral centre. In certain embodiments, the carbon atom to which $R_1$, $R_2$ and $R_3$ are attached is a chiral centre and the chirality remains unchanged throughout the reaction.

The "hydrocarbon group optionally comprising up to 3 heteroatoms selected from O, S, N and F" may, for example, comprise from 1 to 15 carbon atoms. For example, the "hydrocarbon group optionally comprising up to 3 heteroatoms selected from O, S, N and F" may comprise from 2 to 15 carbon atoms. For example, the "hydrocarbon group optionally comprising up to 3 heteroatoms selected from O, S, N and F" may comprise 1, 2, 3, 4, 5, 6, 7, 8, 9, 10, 11, 12, 13, 14 or 15 carbon atoms.

$R_1$, $R_2$ and $R_3$ together with the carbon atom to which they are attached may, for example, form a hydrocarbon group selected from 3-thiabut-2-yl, 2-methyl-3-thiabut-2-yl, 3-thiapent-2-yl, 4-thiapent-2-yl, 2-thiaprop-1-yl, 2-methyl-3-thiapent-2-yl, 3-oxo-3-thiabut-2-yl, 3-oxo-2-methyl-3-thiabut-2-yl, 3-oxo-3-thiapent-2-yl, 4-oxo-4-thiapent-2-yl, 2-oxo-2-thiaprop-1-yl, 3-oxo-2-methyl-3-thiapent-2-yl, but-2-yl, pent-2-yl, but-3-en-2-yl, pent-3-en-2-yl, but-2-en-2-yl, pent-2-en-2-yl, but-1-en-2-yl, pent-1-en-2-yl, 2-methylbut-2-yl, 2-methylpent-2-yl, 2-methylbut-3-en-2-yl, 3-methylbut-2-yl, 3-methylbut-3-en-2-yl, 3-methylbut-2-en-2-yl, 2,3-dimethylbut-2-yl, 2,3-dimethylpent-2-yl, 2,3-dimethylbut-3-en-2-yl, 2,3-dimethylpent-3-en-2-yl, 2-methylpent-3-en-2-yl, prop-2-yl, prop-1-yl, ethyl, cyclopropyl, 1,1-dimethylcycloprop-2-yl, 1-methylcycloprop-2-yl, 1-methylcycloprop-1-yl, 3-thiahex-5-en-2-yl, 2-methyl-3-thiahex-5-en-2-yl, 1-mercaptoeth-1-yl, 2-mercaptoprop-2-yl, 3,3,3-trifluoroprop-2-yl, 2-methyl-3,3,3-trifluoroprop-2-yl, 1-(2-furyl)eth-1-yl, 1-(5-methylfur-2-yl)eth-1-yl, 2-(2-furyl)prop-2-yl, 1-(3-furyl)eth-1-yl, 1-(5-methylfur-3-yl)eth-1-yl, 2-(3-furyl)prop-2-yl, 1-(2-tetrahydrofuryl)eth-1-yl, 2-(2-tetrahydrofuryl)prop-2-yl, 1-(3-tetrahydrofuryl)eth-1-yl, 2-(3-tetrahydrofuryl)prop-2-yl, 1-cyclopropyleth-1-yl, 2-cyclopropylprop-2-yl, 1-cyclobutyleth-1-yl, 2-cyclobutylprop-2-yl, cyclobutyl, cyclopentyl, pent-2-en-3-yl, 1-methoxyprop-1-yl, 1-methoxyeth-1-yl, 1,1,1-trifluorobut-3-yl, 3-thiacyclobut-1-yl, 1-(N-methylamino)eth-1-yl, and 1-(N,N-dimethylamino)eth-1-yl.

$R_1$, $R_2$ and $R_3$ may, for example, each independently be selected from hydrogen, alkyl (including linear alkyl groups (straight chain and branched chain) and cycloalkyl groups), alkenyl, alkoxy, alkyl-C(O)-, alkyl-S-, alkyl-S-alkyl (e.g. alkyl-S-$CH_2$-), alkenyl-S-, alkyl-S(O)-, alkyl-S$(O)_2$-, alkenyl-S(O)-, alkenyl-S$(O)_2$-, -SH, $CF_3$S-, furyl (e.g. 2-furyl or 3-furyl) optionally substituted with alkyl (e.g. furyl optionally substituted with methyl) and fluoro-alkyl.

$R_1$, $R_2$ and $R_3$ may, for example, each independently be selected from hydrogen, linear $C_1$-$C_4$-alkyl, $C_3$-$C_4$-cycloalkyl, $C_2$-$C_3$ alkenyl comprising one or two double bonds, $C_1$-$C_3$-alkoxy, $C_1$-$C_4$-alkyl-C(O)-, $C_1$-$C_4$-alkyl-S-, $C_1$-$C_4$-alkyl-S-$C_1$-$C_4$-alkyl (e.g. $C_1$-$C_4$-alkyl-S-$CH_2$-), $C_1$-$C_4$-alkenyl-S, $C_1$-$C_4$-alkyl-S(O)-, $C_1$-$C_4$-alkyl-S$(O)_2$-, $C_1$-$C_4$-alkenyl-S(O)-, $C_1$-$C_4$-alkenyl-S$(O)_2$-, -SH, $CF_3$S-, furyl (e.g. 2-furyl or 3-furyl) optionally substituted with $C_1$-$C_4$-alkyl (*e.g.* furyl optionally substituted with methyl) and $C_1$-$C_6$-fluoro-alkyl (e.g. difluoromethyl, trifluoromethyl, 2,2,2-trifluoroethyl or difluoroethyl).

$R_1$ may, for example, be selected from hydrogen and $C_1$-$C_4$-alkyl. For example, $R_1$ may be selected from hydrogen and methyl.

$R_2$ may, for example, be selected from hydrogen, $C_1$-$C_4$-alkyl, and $C_2$-$C_5$-alkenyl comprising one or two double bonds. For example, $R_2$ may be selected from hydrogen, $C_1$-$C_2$-alkyl, and $C_2$-$C_3$-alkenyl. For example, $R_2$ may be methyl.

$R_3$ may, for example, be selected from $C_1$-$C_4$ alkyl, $C_2$-$C_5$ alkenyl comprising one or two double bonds, $C_1$-$C_3$ alkoxy, $C_1$-$C_4$-alkyl-C(O)-, $C_1$-$C_4$-alkyl-S-, $C_1$-$C_4$-alkyl-$SCH_2$, $C_1$-$C_4$-alkenyl-S-, $C_1$-$C_4$-alkyl-S(O)-, $C_1$-$C_4$-alkyl-S$(O)_2$-, $C_1$-$C_4$-alkenyl-S(O)-, $C_1$-$C_4$-alkenyl-S$(O)_2$-, - SH, $CF_3$S-, cyclopropyl, cyclobutyl, furyl (e.g. 2-furyl or 3-furyl) optionally substituted with methyl, and $C_1$-$C_6$-fluoro-alkyl (*e.g.* difluoromethyl, trifluoromethyl, 2,2,2-trifluoroethyl or difluoroethyl). For example, $R_3$ may be selected from $C_1$-$C_4$ alkyl, $C_2$-$C_5$ alkenyl comprising one or two double bonds, and $C_1$-$C_4$-alkyl-S-. For example, $R_3$ may be selected from $C_1$-$C_2$ alkyl, $C_2$-$C_3$ alkenyl comprising one double bond, and $C_1$-$C_2$-alkyl-S-. For example, $R_3$ may be selected from ethyl, ethenyl, and -$SCH_3$.

For example, $R_1$ may be selected from hydrogen and $C_1$-$C_4$-alkyl, $R_2$ may be selected from hydrogen,

$C_1$-$C_4$-alkyl, and $C_2$-$C_5$-alkenyl comprising one or two double bonds, and $R_3$ may be selected from $C_1$-$C_4$ alkyl, $C_2$-$C_5$ alkenyl comprising one or two double bonds, $C_1$-$C_3$ alkoxy, $C_1$-$C_4$-alkyl-C(O)-, $C_1$-$C_4$-alkyl-S-, $C_1$-$C_4$-alkyl-$SCH_2$-, $C_1$-$C_4$-alkenyl-S-, $C_1$-$C_4$-alkyl-S(O)-, $C_1$-$C_4$-alkyl-$S(O)_2$-, $C_1$-$C_4$-alkenyl-S(O)-, $C_1$-$C_4$-alkenyl-$S(O)_2$-, -SH, $CF_3S$-, cyclopropyl, cyclobutyl, furyl (*e.g.* 2-furyl or 3-furyl) optionally substituted with methyl, and $C_1$-$C_6$-fluoro-alkyl (e.g. difluoromethyl, trifluoromethyl, 2,2,2-trifluoroethyl or difluoroethyl).

For example, $R_1$ may be selected from hydrogen and methyl, $R_2$ may be selected from hydrogen, $C_1$-$C_2$-alkyl, and $C_2$-$C_3$-alkenyl, and $R_3$ may be selected from $C_1$-$C_4$ alkyl, $C_2$-$C_5$ alkenyl comprising one or two double bonds, $C_1$-$C_3$ alkoxy, $C_1$-$C_4$-alkyl-C(O)-, $C_1$-$C_4$-alkyl-S-, $C_1$-$C_4$-alkyl-$SCH_2$-, $C_1$-$C_4$-alkenyl-S-, $C_1$-$C_4$-alkyl-S(O)-, $C_1$-$C_4$-alkyl-$S(O)_2$-, $C_1$-$C_4$-alkenyl-S(O)-, $C_1$-$C_4$-alkenyl-$S(O)_2$-, -SH, $CF_3S$-, cyclopropyl, cyclobutyl, furyl (e.g. 2-furyl or 3-furyl) optionally substituted with methyl, and $C_1$-$C_6$-fluoro-alkyl (*e.g.* difluoromethyl, trifluoromethyl, 2,2,2-trifluoroethyl or difluoroethyl).

For example, $R_1$ may be selected from hydrogen and methyl, $R_2$ may be $C_1$-$C_2$-alkyl and $R_3$ may be selected from $C_1$-$C_4$ alkyl, $C_2$-$C_5$ alkenyl comprising one or two double bonds, and $C_1$-$C_4$-alkyl-S-.

For example, $R_1$ may be selected from hydrogen and methyl, $R_2$ may be methyl, and $R_3$ may be ethyl, ethenyl or -$SCH_3$.

The acid chloride of formula (Ia) or the acid anhydride of formula (Ib) may, for example, be added to the reaction mixture neat or as a solution in the solvent, for example as a solution in the organic solvent having a boiling temperature ranging from about 50°C to about 160°C.

The compound of formula (III) may, for example, be added to the reaction mixture neat or as a solution in the solvent (e.g. the organic solvent having a boiling temperature ranging from about 50°C to about 160°C). This may, for example, assist in reducing exposure to the irritant compounds.

The reaction of pipecolic acid with the acid chloride of formula (Ia) or the acid anhydride of formula (Ib) may preferably take place in the presence of a base. The base deprotonates the carboxylic acid of the pipecolic acid. Therefore, any base suitable to deprotonate the pipecolic acid may be used. The base may, for example, be an inorganic or an organic base.

Examples of inorganic bases include metal phosphates, metal hydroxides, metal carbonates, metal bicarbonates and combinations thereof.

Examples of organic bases include alkylamines such as tributylamine and alkanolamines such as triethanolamine, and combinations thereof.

The base may, for example, be a metal phosphate, a metal hydroxide, a metal carbonate, a metal bicarbonate or a combination thereof. The metal may, for example, be an alkali metal or an alkali earth metal. $B^+$ of formula (II) may, for example, be the metal cation of the metal phosphate, metal hydroxide or metal carbonate.

In particular, the base may be a metal hydroxide, for example selected from sodium hydroxide and potassium hydroxide.

Further, the base may also neutralize any acid formed as a result of the reaction of the pipecolic acid with a compound of formula (Ia), or (Ib), for example the base may neutralize the HCl formed as a result of the reaction of the pipecolic acid with the acid chloride of formula (Ia). Therefore, at least about two equivalents of base to acid chloride of formula (Ia) may be used (one equivalent to deprotonate the pipecolic acid and one equivalent to neutralize the acid formed (e.g. HCl which is formed when pipecolic acid is reacted with an acid chloride of formula (Ia)). For example from about two equivalents to about four equivalents or from about two equivalents to about three equivalents of base to acid chloride of formula (Ia) may be used.

At least about one equivalent of the acid chloride of formula (Ia) or the acid anhydride of formula (Ib) to the pipecolic acid may, for example, be used.

The compound of formula (II) has the following chemical structure:

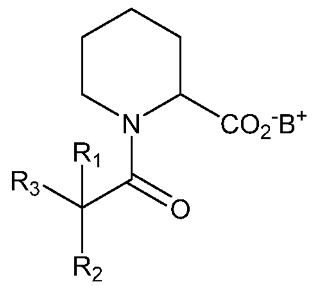

Formula (II)

wherein $R_1$, $R_2$ and $R_3$ are as defined in relation to the acid chloride of formula (Ia) and the acid anhydride of formula (Ib), and $B^+$ is a cation provided by the base. For example, $B^+$ may be $Na^+$ or $K^+$ when sodium hydroxide or potassium hydroxide respectively are used as the base.

The reaction of the pipecolic acid with the acid chloride of formula (Ia) or the acid anhydride of formula (Ib) may, for example, take place in the presence of a phase transfer catalyst. In particular, the reaction of the pipecolic acid with the acid chloride of formula (Ia) or the acid anhydride of formula (Ib) to form the compound of formula (II) may take place in the presence of a phase transfer catalyst when the solvent (e.g. the organic solvent having a boiling point ranging from about 50°C to about 160°C) is a non-polar solvent, or when the solvent is a mixture of organic solvent and water. The use of a phase transfer catalyst may, for example, act to increase the yield of the reaction and/or reduce the hydrolysis of the acid chloride of formula (Ia) or the acid anhydride of formula (Ib).

A "phase transfer catalyst" refers to a substance that facilitates the migration of a substance from one phase into another. The phase transfer catalyst may, for example, act to facilitate the migration of the acid chloride of formula (Ia) or the acid anhydride of formula (Ib) into a water phase where it reacts with pipecolic acid, particularly where the solvent (e.g. the organic solvent having a boiling point ranging from about 50°C to about 160°C) is a non-polar solvent.

Certain solvents or bases used in the *in-situ* reaction may, for example, also act as phase transfer catalysts. For example, partially water soluble solvents such as methyltetrahydrofuran (Me-THF) may act as phase transfer catalysts. Metal phosphates or metal carbonates may, for example, act as phase transfer catalysts. Where a solvent or base used in the *in-situ* reaction acts as a phase transfer catalyst, it may not be necessary to use an additional phase transfer catalyst. Therefore, where a metal phosphate or a metal carbonate is used as a base, it may not be necessary to use an additional phase transfer catalyst.

The phase transfer catalyst may, for example, be a metal halide (e.g. potassium iodide or sodium iodide).

The phase transfer catalyst may, for example, be a quaternary ammonium salt ($NR_4^+$ where R is an alkyl or aryl group) or an organic phosphonium salt ($PR_4^+$ where R is hydrogen, alkyl, aryl or halide).

Examples of quaternary ammonium salts that may be used as phase transfer catalysts include benzyltriethylammonium salts (e.g. benzyltriethylammonium chloride), methyltricaprylammonium salts (e.g. methyltricaprylammonium chloride), methyltributylammonium salts (e.g. methyltributylammonium chloride), methyltrioctylammonium salts (e.g. methyltrioctylammonium chloride), and tetra-n-butylammonium salts. An example of phosphonium salts that may be used as phase transfer catalysts is hexadecyltributylphosphonium salts (e.g. hexadecyltributylphosphonium bromide).

For example, the phase transfer catalyst may be a tetra-n-butylammonium salt, for example a tetra-n-butylammonium halide, for example tetra-n-butylammonium bromide (TBAB).

The reaction of the pipecolic acid with the acid chloride of formula (Ia) or the acid anhydride of formula (Ib) to form the compound of formula (II) may take place at any suitable pH, for example a pH ranging from about 7.0 to about 15.0 or from about 8.0 to about 14.0 or from about 9.0 to about 14.0 or from about 10.0 to about 15.0.

The reaction of the pipecolic acid with the acid chloride of formula (Ia) or the acid anhydride of formula (Ib) to form the compound of formula (II) may, for example, take place at a pH of at least about 12.5. For example, the reaction of the pipecolic acid with the acid chloride of formula (Ia) or the acid anhydride of formula (Ib) may take place at a pH of at least about 13.0. The reaction of the pipecolic acid with the acid chloride of formula (Ia) or the acid anhydride of formula (Ib) to form the compound of formula (II) may, for example, take place at a pH up to about 14.5. For example, the reaction of the pipecolic acid with the acid chloride of formula (Ia) or the acid anhydride of formula (Ib) to form the compound of formula (II) may, for example, take place at a pH up to about 14.0 or up to about 13.5. For example, the reaction of the pipecolic acid with the acid chloride of formula (Ia) or the acid anhydride of formula (Ib) to form the compound of formula (II) may, for example, take place at a pH ranging from about 12.5 to about 14.5 or from about 12.5 to about 13.5. The pH of the reaction mixture may, for example, be maintained throughout the reaction of the pipecolic acid with the acid chloride of formula (Ia) or the acid anhydride of formula (Ib) to form the compound of formula (II). The pH of the reaction mixture may, for example, be maintained by controlling the time and amount of base added to the reaction mixture. This may, for example, involve continuous monitoring of the pH of the reaction mixture using a pH electrode. Controlling the pH of the reaction mixture during the reaction of the pipecolic acid with the acid chloride of formula (Ia) or the acid anhydride of formula (Ib) may, for example, help to minimize the hydrolysis of the acid chloride or acid anhydride.

The reaction of the pipecolic acid with the acid chloride of formula (Ia) or the acid anhydride of formula (Ib) to form the compound of formula (II) may, for example, take place at a temperature of at least about -10°C. For example, the reaction of the pipecolic acid with the acid chloride of formula (Ia) or the acid anhydride of formula (Ib) to form the compound of formula (II) may take place at a temperature of at least about -5°C or at least about 0°C. For example, the reaction of the pipecolic acid with the acid chloride of formula (Ia) or the acid anhydride of formula (Ib) to form the compound of formula (II) may take place at a temperature up to about 40°C or up to about 35°C or up to about 30°C or up to about 25°C or up to about 20°C or up to about 15°C or up to about 10°C. For example, the reaction of the pipecolic acid with the acid chloride of formula (Ia) or the acid anhydride of formula (Ib) to form the compound of formula (II) may take place at a temperature ranging from about -10°C to about 40°C or from about -5°C to about 30°C or from about 0°C to about 20°C or from about 0°C to about 15°C or from about 0°C to about 5°C.

The reaction of the pipecolic acid with the acid chloride of formula (Ia) or the acid anhydride of formula (Ib) to form the compound of formula (II) may, for example take place at a temperature lower than the temperature at which

the pipecolic acid and/or the acid chloride of formula (Ia) or the acid anhydride of formula (Ib) decomposes.

The reaction of the pipecolic acid with the acid chloride of formula (Ia) or the acid anhydride of formula (Ib) to form the compound of formula (II) may, for example, take place for a period of time ranging from about 30 seconds to about 5 hours. For example, the reaction of the pipecolic acid with the acid chloride of formula (Ia) or the acid anhydride of formula (Ib) to form the compound of formula (II) may, for example, take place for a period of time ranging from about 30 seconds to about 1 hour or from about 1 minute to about 30 minutes or from about 1 minute to about 15 minutes or from about 1 minute to about 5 minutes. The reaction of the pipecolic acid with the acid chloride of formula (Ia) or the acid anhydride of formula (Ib) to form the compound of formula (II) may, for example, take place until the reaction is complete.

*Step 2 - Conversion of a compound of formula (II) to a compound of formula (IV)*

The *in-situ* method described herein may further comprise reacting the compound of formula (II) as described herein with a compound of formula (III) to form a compound of formula (IV).

The compound of formula (III) has the following chemical structure:

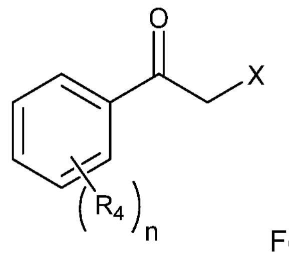

Formula (III),

wherein the phenyl group of the compound of formula (III) is substituted with n $R_4$ substituents, wherein n is zero, one, two, three, four or five,

each $R_4$ substituent (where present) is independently selected from halogen (*e.g.* F, Cl or Br), cyano (C=N), nitro ($-NO_2$), linear $C_1$-$C_6$-alkyl (straight chain or branched) optionally comprising up to 5 halogen atoms (*e.g.* up to 5 F atoms) (*e.g.* $CH_3$, $CF_3$ or $CHF_2$), $C_2$-$C_6$-alkenyl (*e.g.* comprising one or two double bonds) (*e.g.* $-CH=CH_2$), $C_1$-$C_6$-alkoxy optionally comprising up to 3 halogen atoms (*e.g.* up to 3 F atoms) (*e.g.* $-OCH_3$, $-OCF_3$, $-OCHF_2$, $-OCH_2F$), $C_1$-$C_3$-alkoxy-$C_1$-$C_3$-alkyl (*e.g.* 2-methoxy-ethyl), and $C_3$-$C_7$-cycloalkyl (*e.g.* cyclopropyl or cyclobutyl), and X is a halogen.

The compound of formula (III) may, for example, be obtained commercially or may, for example, be made by Friedel-Crafts acylation of benzene or substituted benzene using chloroacetyl chloride with an aluminum chloride catalyst, or by chlorination of the corresponding acetophenone with sulfuryl chloride ($SO_2Cl_2$) or 1,3-dichloro-5,5-dimethylhydantoin or N-chlorosuccinimide.

For example, $R_4$ may be a linear $C_1$-$C_6$-alkyl (straight chain or branched) optionally comprising up to 5 halogen atoms (*e.g.* up to 5 F atoms) (*e.g.* $CH_3$, $CF_3$ or $CHF_2$). For example, $R_4$ may be methyl.

For example, the phenyl group of the compound of formula (III) may be substituted with one or two $R_4$ substituents. For example, the phenyl group of the compound of formula (III) may be substituted with one $R_4$ substituent which is methyl.

For example, the phenyl group of the compound of formula (III) may be substituted with five $R_4$ substituents. For example, the phenyl group of the compound of formula (III) may be substituted with five $R_4$ substituents, all of which are methyl.

For example, X may be chlorine, bromine or iodine. For example, X may be chlorine.

The compound of formula (III) may, for example, be added to the reaction mixture neat or as a solution in the solvent (e.g. the organic solvent having a boiling temperature ranging from about 50°C to about 160°C). This may, for example, assist in maintaining a stirrable mixture throughout the reaction of the compound of formula (II) with the compound of formula (III) to make the compound of formula (IV). The use of a solution may, for example, assist in reducing exposure to the irritant compounds.

For example, the reaction of the compound of formula (II) with the compound of formula (III) may be performed at a temperature ranging from about 50°C to about 160°C, for example from about 60°C to about 150°C, for example from about 80°C to about 130°C, for example from about 90°C to about 120°C, for example from about 100°C to about 110°C.

The compound of formula (IV) has the following chemical structure:

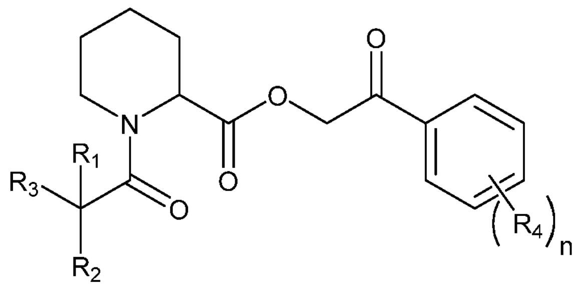

Formula (IV),

wherein $R_1$, $R_2$ and $R_3$ are as defined herein in relation to the compound of formula (Ia), (Ib) and (II), and $R_4$ and n are as defined herein in relation to the compound of formula (III).

The reaction of the compound of formula (II) with the compound of formula (III) to form the compound of formula (IV) may, for example, take place in the presence of a phase transfer catalyst. In particular, the reaction of the compound of formula (II) with the compound of formula (III) to form the compound of formula (IV) may take place in the presence of a phase transfer catalyst when the solvent (e.g. organic solvent having a boiling point ranging from about 50°C to about 160°C) is a non-polar solvent.

The phase transfer catalyst may already be present in the reaction mixture where the reaction of the pipecolic acid with the acid chloride of formula (Ia) or the acid anhydride of formula (Ib) to form the compound of formula (II) took place in the presence of the phase transfer catalyst. Therefore, it may not be necessary to add further phase transfer catalyst to the reaction mixture for the reaction of the compound of formula (II) with the compound of formula (III) to form the compound of formula (IV).

The phase transfer catalyst may, for example, facilitate the migration of compound of formula (III) into a water phase where it reacts with the compound of formula (II) to form a compound of formula (IV). The compound of formula (IV) may then migrate into an organic layer (e.g. organic solvent having a boiling point ranging from about 50°C to about 160°C).

The phase transfer catalyst may, for example, be as defined in relation to step 1 herein. For example, the phase transfer catalyst may be an ammonium salt such as tetra-n-butylammonium bromide (TBAB).

It may, for example, not be necessary to add any further base to the reaction mixture during the reaction of the compound of formula (II) with the compound of formula (III) to form the compound of formula (IV). This may, for example, be because the carboxylic acid of the compound of formula (II) is already deprotonated.

The reaction of the compound of formula (II) with the compound of formula (III) may, for example, be performed at a temperature and pressure to obtain reflux of the reaction mixture. The reflux temperature of the reaction mixture may, for example, be different to (e.g. lower than) the reflux temperature of the solvent (e.g. organic solvent having a boiling point ranging from about 50°C to about 160°C) due to the presence of other components in the mixture such as water, which may, for example, result in the formation of an azeotrope.

The reaction of the compound of formula (II) with the compound of formula (III) may, for example, be performed at a temperature lower than the temperature at which the compound of formula (II) and/or the compound of formula (III) decomposes.

For example, the reaction of the compound of formula (II) with the compound of formula (III) may be performed at a temperature equal to or greater than about 50°C, for example equal to or greater than about 60°C, for example equal to or greater than about 70°C, for example equal to or greater than about 80°C.

For example, the reaction of the compound of formula (II) with the compound of formula (III) may be performed at a temperature equal to or less than about 160°C, for example equal to or less than about 150°C, for example equal to or less than about 140°C, for example equal to or less than about 130°C, for example equal to or less than about 120°C, for example equal to or less than about 110°C, for example equal to or less than about 100°C.

For example, the reaction of the compound of formula (II) with the compound of formula (III) may be performed at a temperature ranging from about 50°C to about 160°C, for example from about 60°C to about 120°C, for example from about 70°C to about 100°C.

The reaction of the compound of formula (II) with the compound of formula (III) may, for example, be performed at any suitable pH provided the carboxylic acid is deprotonated, for example a pH equal to or greater than about 7.0 or equal to or greater than about 8.0 or equal to or greater than about 9.0 or equal to or greater than about 10.0.

The reaction of the compound of formula (II) with the compound of formula (III) may, for example, be performed at a pH equal to or greater than about 12.0, for example equal to or greater than about 12.5. The reaction of the compound of formula (II) with the compound of formula (III) may, for example, be performed at a pH equal to or less than about 14.0, for example equal to or less than about 13.5. For example, the reaction of the compound of formula (II) with the

compound of formula (III) may be performed at a pH ranging from about 12.0 to about 14.0 or from about 12.5 to about 13.5.

The reaction of the compound of formula (II) with the compound of formula (III) to form the compound of formula (IV) may, for example, take place for a period of time ranging from about 30 seconds to about 5 hours. For example, the reaction of the compound of formula (II) with the compound of formula (III) to form the compound of formula (IV) may take place for a period of time ranging from about 30 seconds to about 1 hour or from about 1 minute to about 30 minutes or from about 1 minute to about 15 minutes or from about 1 minute to about 5 minutes. The reaction of the compound of formula (II) with the compound of formula (III) to form the compound of formula (IV) may, for example, take place until the reaction is complete. This may, for example, be determined by gas chromatography analysis.

In case that an organic solvent is used, water may, for example, be removed from the reaction mixture after the reaction of the compound of formula (II) with the compound of formula (III) to form the compound of formula (IV) and before the reaction of the compound of formula (IV) with the ammonium source to form the compound of formula (V). One advantage of removing the water is, to increase the reaction temperature which speeds up the reaction. For example, water may be removed from the reaction mixture after the reaction of the compound of formula (II) with the compound of formula (III) to form the compound of formula (IV) is complete and before the reaction of the compound of formula (IV) with the ammonium source to form the compound of formula (V). Water may, for example, be removed by any suitable method. For example, water may be removed by azeotropic distillation. The product of the reaction (the compound of formula (IV)) may remain in an organic layer (e.g. comprising the organic solvent having a boiling point ranging from about 50°C to about 160°C). This may, for example, advantageously allow step 3 to be performed at a higher temperature, for example by performing reflux at a higher temperature.

*Step 3 - Conversion of a compound of formula (IV) to a compound of formula (V)*

The *in-situ* method described herein further comprises reacting the compound of formula (IV) as described herein with an ammonium source to form a compound of formula (V).

The compound of formula (V) has the following chemical structure:

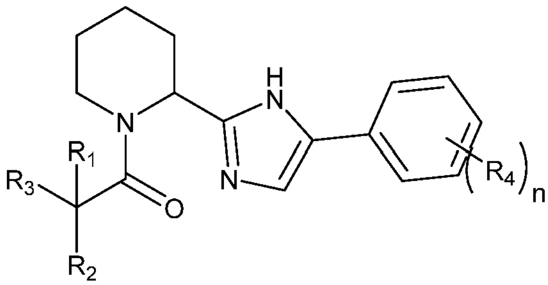

Formula (V),

wherein $R_1$, $R_2$, $R_3$, $R_4$ and n are as defined in relation to the compounds of formula (Ia), (Ib), (II), (III), and (IV) herein.

The compound of formula (V) may, for example, be 2-methyl-1-(2-(5-(p-tolyl)-1H-imidazol-2-yl)piperidin-1-yl)butan-1-one (including (2S)-2-methyl-1-(2-(5-(p-tolyl)-1H-imidazol-2-yl)piperidin-1-yl)butan-1-one, or racemic mixture), 2-(methylthio)-1-(2-(5-(p-tolyl)-1H-imidazol-2-yl)piperidin-1-yl)propan-1-one, 2-methyl-2-(methylthio)-1-(2-(5-(p-tolyl)-1H-imidazol-2-yl)piperidin-1-yl)propan-1-one, or 2,2-dimethyl-1-(2-(5-(p-tolyl)-1H-imidazol-2-yl)piperidin-1-yl)but-3-en-1-one.

The ammonium source may, for example, be any ammonium source suitable to convert the compound of formula (IV) to the compound of formula (V).

The ammonium source may, for example, be an ammonium carboxylate such as ammonium acetate or ammonium formate. The ammonium source may, for example, be a mixture of ammonia and a carboxylic acid ($RCO_2H$ wherein R is hydrogen or an alkyl group). For example, the ammonium source may be ammonium acetate or a mixture of ammonia and acetic acid.

Suitable ammonium sources such as ammonium acetate are available commercially.

The ammonium source may, for example, be added to the reaction mixture as a solution in water. The solution may, for example, be heated prior to adding it to the reaction mixture in order to keep the ammonium source dissolved at a high concentration. Adding the ammonium source in solution with water may, for example, help to prevent accumulation of unreacted ammonium source.

Equal to or less than about five equivalents of the ammonium source to the compound of formula (IV) may, for example, be used. For example, equal to or less than about four equivalents or equal to or less than about three equivalents of the ammonium source to the compound of formula (IV) may be used. For example, equal to or greater

than about one equivalent or equal to or greater than about two equivalents of the ammonium source to the compound of formula (IV) may be used.

The ammonium source may, for example, be added to the reaction mixture in an aqueous solution or may be added to the reaction mixture in its natural form (i.e. not in solution), for example as a solid. In certain embodiments, the ammonium source may be added to the reaction mixture in its natural form, for example as a solid, which may, for example, be advantageous in that it minimizes the amount of water that is introduced into the reaction.

The ammonium source may, for example, be added to the reaction mixture in portions (i.e. in separate batches as opposed to all in one). This may, for example, assist in making the reaction more efficient, for example because the proportion of water in the reaction mixture due to the presence of water in the solution of the ammonium source is minimized. For example, the ammonium source may be added to the reaction mixture in at least two or three or four portions. For example, where the ammonium source is added to the reaction mixture, it may be added in at least two or at least three portions, for example from two to six portions or from three to six portions or from two to four portions or from three to four portions. For example, where the ammonium source is added to the reaction mixture in an aqueous solution, it may be added in a dropwise fashion.

The reaction of the compound of formula (IV) with the ammonium source to form the compound of formula (V) may, for example, be performed at a temperature and pressure to obtain reflux of the reaction mixture. The reflux temperature of the reaction mixture may, for example, be different to (e.g. lower than) the reflux temperature of the solvent (e.g. organic solvent having a boiling point ranging from about 50°C to about 160°C) due to the presence of other components in the mixture such as water, which may, for example, result in the formation of an azeotrope.

The reaction of the compound of formula (IV) with the ammonium source to form the compound of formula (V) may, for example, be performed at a temperature lower than the temperature at which the compound of formula (IV) and/or the ammonium source decomposes.

For example, the reaction of the compound of formula (IV) with the ammonium source to form the compound of formula (V) may be performed at a temperature equal to or greater than about 50°C, for example equal to or greater than about 60°C, for example equal to or greater than about 70°C, for example equal to or greater than about 80°C, for example equal to or greater than about 90°C, for example equal to or greater than about 100°C.

For example, the reaction of the compound of formula (IV) with the ammonium source to form the compound of formula (V) may be performed at a temperature equal to or less than about 160°C, for example equal to or less than about 150°C, for example equal to or less than about 140°C, for example equal to or less than about 130°C, for example equal to or less than about 120°C, for example equal to or less than about 110°C.

The reaction of the compound of formula (IV) with the ammonium source to form the compound of formula (V) may, for example, be performed at a pH equal to or greater than about 1.0, for example equal to or greater than about 2.0. The reaction of the compound of formula (IV) with the ammonium source to form the compound of formula (V) may, for example, be performed at a pH equal to or less than about 14.0, for example equal to or less than about 12.0 or equal to or less than about 10.0 or equal to or less than about 8.0 or equal to or less than about 7.0 or equal to or less than about 6.0. For example, the reaction of the compound of formula (IV) with the ammonium source to form the compound of formula (V) may be performed at a pH ranging from about 1.0 to about 14.0 or from about 1.0 to about 8.0 or from about 1.0 to about 7.0 or from about 2.0 to about 6.0. This may, for example, be due to an accumulation of acid (e.g. acetic acid) produced during step 3 of the reaction.

The reaction of the compound of formula (IV) with the ammonium source to form the compound of formula (V) may, for example, take place for a period of time ranging from about 30 minutes to about 10 hours. For example, the reaction of the compound of formula (IV) with the ammonium source to form the compound of formula (V) may take place for a period of time ranging from about 1 hour to about 8 hours or from about 2 hours to about 7 hours. The reaction of the compound of formula (IV) with the ammonium source to form the compound of formula (V) may, for example, take place until the reaction is complete. This may, for example, be determined by gas chromatography analysis.

In case that an organic solvent is used, water may, for example, be removed from the reaction mixture during the reaction of the compound of formula (IV) with the ammonium source to form the compound of formula (V). For example, water may be continuously removed from the reaction mixture during the reaction of the compound of formula (IV) with the ammonium source to form the compound of formula (V). The water may, for example, be removed using a Dean-Stark water separator. The product of the reaction (the compound of formula (V)) may remain in an organic layer (e.g. comprising the organic solvent having a boiling point ranging from about 50°C to about 160°C).

*Further Steps*

The reaction of the compound of formula (IV) with the ammonium source to form the compound of formula (V) may, for example, be followed by neutralizing the layer comprising the compound of formula (V) (e.g. organic layer which may comprise the organic solvent having a boiling point ranging from about 50°C to about 160°C) and/or by washing with water. The washing with water may, for example, occur after the layer comprising the compound of formula (V)

(e.g. organic layer) is neutralized.

The compound of formula (V) may, for example, be isolated (e.g. isolated from an organic layer which may comprise the organic solvent having a boiling point ranging from about 50°C to about 160°C) by any suitable method, for example by crystallization. For example, the compound of formula (V) may be crystallized directly from the solvent (for example, toluene, or Me-THF), which may be used as the solvent throughout the synthesis, thus providing the advantage of not needing to change the solvent throughout the entire synthesis and crystallization steps. Or the compound of formula (V) may be crystallized by switching the solvent. The compound of formula (V) may then be subjected to further purification steps.

Depending on the solvent used for the reaction a solvent switch might be an advantage to obtain an olfactory pure quality and/or higher recovery rates of the compound of formula (V). Suitable solvents for crystallisation may be selected from but not limited to ethyl acetate, butyl acetate, isopropyl acetate, isobutyl acetate, methyl isobutyl ketone, and isopropanol, and mixtures such as heptane / isopropanol; heptane / ethanol, or methyl tert-butyl ether / ethyl acetate.

*Uses of the Compounds of Formula (V)*

There is further provided herein the compounds of formula (V) which may, for example, be obtained by or obtainable by the methods described herein.

The compounds of formula (V) may, for example, be used in a flavor composition. In particular, the compounds of formula (V) may be used as a cooling agent in a flavor composition.

There is therefore also provided herein a flavor composition comprising a compound of formula (V) as described herein.

The flavor compositions described herein may, for example, be incorporated into any consumer product that contacts a mucous membrane. For example, the consumer product may be a food product, a beverage, chewing gum, a tobacco product, a tobacco replacement product, a dental care product, a personal care product (including lip care products), or a sexual health and intimate care product.

There is therefore also provided herein a consumer product comprising a flavor composition as described herein.

The methods are now further described with reference to the following non-limiting examples, which describe particular embodiments.

Example 1: One pot procedure for the preparation of 2-methyl-1-(2-(5-(p-tolyl)-1H-imidazol-2-vl)piperidin-1-vl)butan-1-one (compound of formula (V))

A 10 l reactor was flushed with nitrogen and charged at room temperature with pipecolic acid (DL-pipecolinic acid, Xiamen Synress Import and Export Co., Ltd, (800 g, 6.2 mol)) and tetrabutylammonium bromide (100 g, 0.3 mol). Water (1630 g), toluene (1127 g) and sodium hydroxide (32%, 1664 g, 13.3 mol) were successively added to the stirred solution (102 rpm). The stirring speed was increased (186 rpm) and the mixture was cooled to 5 °C ($T_j$: -15 °C). 2-Methylbutanoyl chloride (747 g, 6.2 mol) was added dropwise over the period of 2 hours, keeping the temperature of the reaction mixture between 3 °C and 5 °C. Following the addition, the mixture was stirred for one hour before the temperature was raised to 45 °C and solid 2-chloro-1-(p-tolyl)ethan-1-one (1077 g, 6.3 mol) was added in portions over 15 minutes. The mixture was then heated to 90 °C ($T_j$: 110 °C) for 1 hour and 15 minutes, after which GC analysis of the mixture showed complete conversion of 1-(2-methylbutanoyl)piperidine-2-carboxylic acid to 2-oxo-2-(p-tolyl)ethyl 1-(2-methylbutanoyl)piperidine-2-carboxylate. The reaction mixture was then cooled to 50 °C ($T_j$: 50 °C), the stirring was stopped and the water layer was removed from the turbid-orange organic layer through the bottom valve. Toluene (607 g) was added to the mixture remaining in the reactor. The solution was stirred (160 rpm) and heated to reflux (89-114 °C, $T_j$: 145 °C) while removing the water azeotropically. After all the water was removed and the reflux temperature had reached the final temperature (114 °C), a first portion of ammonium acetate (477 g, 6.2 mol) was added. The reaction mixture was stirred at reflux for 45 minutes and the water produced by the reaction was removed azeotropically. Another portion of ammonium acetate (477 g, 6.2 mol) was added and finally after 3 hours reaction time, the last portion ammonium acetate was added (477 g, 6.2 mol) and stirring at reflux was continued for 70 minutes until analysis by GC showed full conversion. The reaction mixture was cooled to 50 °C and water (500 g) and sodium hydroxide (2M, 1000 g) were added to the stirred mixture. Then the pH of the mixture was adjusted to pH 7 by adding sodium hydroxide (2M, 2700 g). The product solution was heated to reflux again to azeotropically dry the solution (Tj: 145 °C) and then the solution was cooled to ca. 0 °C (Tj: 0 °C) and stirring (70 rpm) was continued for 24 hours. The fine crystals that formed in the red mixture were removed by filtration of the mass through a 4l Buchner funnel and then washed twice with cold methyl-t-butylether (4 °C, 1000 ml).

The solid product was dried in vacuum to give 2-methyl-1-(2-(5-(p-tolyl)-1H-imidazol-2-yl)piperidin-1-yl)butan-1-one (838 g, 2.5 mol, 40% yield) as a white solid. M.p.: 156.3 °C. GC/MS (EI): m/z (%): 325 (10) [M+], 268 (2), 240 (100), 224 (3), 185 (10), 159 (2), 142 (1), 84 (2), 57 (4). $^1$H NMR (500 MHz, DMSO-d6, 413K, mixture of stereoisomers

and tautomers) δ = 11.32 (br s, 1H), 7.61(br m, 2H), 7.28 (br s, 1H), 7.14 (br m, 2H), 5.63 (br m, 1H), 4.06 (br m, 1H), 3.29 (br m, 1H), 2.76 (m, 1H), 2.31 (s, 3H), 2.23 (br m, 1H), 1.83 - 1.60 (m, 5H), 1.49 - 1.33 (m, 2H), 1.09 - 1.05 (2d, *J* = 6.8 Hz, 3H), 0.91 - 0.85 (2t, *J* = 7.4 Hz, 3H) ppm. $^{13}$C NMR (125 MHz, DMSO-d6, 413K, mixture of stereoisomers and tautomers, shifts extracted from HSQC & HMBC experiments) δ 174.4 (s), 146.8 (s), 139.6 (s), 134.3 (s), 132.1 (s), 128.1 (2d), 123.7 (2d), 110.9 (d), 47.5 ( d), 40.1 (t), 35.6 (d), 27.6 (t), 26.0 (t), 24.7 (t), 19.8 (q), 19.0 (t), 16.3 (q), 10.5 (q) ppm.

A crystalline form of 2-methyl-1-(2-(5-(p-tolyl)-1H-imidazol-2-yl)piperidin-1-yl)butan-1-one characterized by main peaks in its powder X-ray diffraction pattern obtained using copper K-beta, radiation at 2-theta (deg) 8.93, 9.59, 11.66, 12.44, 12.95, 14.53, 15.91, 17.13, 17.89, 18.43, 19.21, 19.51, 19.90, 20.76, 22.59, 25.06, 27.03, 27.77, 28.81, 29.79, 32.08.

Example 2: (2S)-2-methyl-1-(2-(5-(p-tolyl)-1H-imidazol-2-vl)piperidin-1-vl)butan-1-one

Prepared from (S)-methylbutanoyl chloride by the disclosed procedure. $[\alpha]_{26}^{589}$ +0.162 (*c* 1.114, EtOH). The spectroscopic data is identical to that of the racemic product in Example 1.

Example 3: 2-(methylthio)-1-(2-(5-(p-tolyl)-1H-imidazol-2-vl)piperidin-1-vl)propan-1-one

Prepared from 2-(methylthio)propanoyl chloride by the disclosed procedure.

GC/MS (EI): *m/z* (%): 357 (5) [$M^+$], 342(7), 268 (11), 240 (100), 185 (11), 159 (5), 117 (4), 89 (10). $^1$H NMR (500 MHz, DMSO-$d_6$, 413K, mixture of stereoisomers and tautomers) $\delta$ 11.56-11.06 (br m, 1H), 7.69 - 7.43 (br m, 2H), 7.34 - 7.24 (br m, 1H), 7.23 - 7.07 (br m, 2H), 5.69 & 5.58 (2 br m, 1H), 4.04 (br m, 1H), 3.91 - 3.87 (br m, 1H), 3.51 - 3.13 (br m, 1H), 2.37 - 2.27 (br m, 1H), 2.32 (br s, 3H), 2.09 & 2.08 (2 br s, 3H), 1.91 - 1.41 (br m, 5H), 1.44 & 1.42 (2 br d, *J* = 6.7 Hz, 3H) ppm. $^{13}$C NMR (125 MHz, DMSO-$d_6$, T = 413K, mixture of stereoisomers and tautomers, shifts extracted from HSQC & HMBC experiments) $\delta$ 169.7 (s), 146.4 (s), 137.7 (s), 134.2 (s), 131.4 (s), 128.0 (2d), 123.7 (2d), 110.9 (d), 47.5 (d), 40.8 (t), 37.7 (d), 27.4 (t), 24.3 (t), 19.7 (q), 18.8 (t), 17.0 (q), 11.0 (q)ppm.

Example 4: One pot procedure for the preparation of 2-methyl-1-(2-(5-(p-tolyl)-1H-imidazol-2-yl)piperidin-1-yl)butan-1-one (compound of formula (V))

A 5 l reactor was flushed with nitrogen and charged at room temperature with pipecolic acid (DL-pipecolinic acid, Xiamen Synress Import and Export Co., Ltd, (400 g, 3.1 mol)), potassium phosphate (98%, 738 g, 3.4 mol) and potassium hydroxide (85%, 184 g, 2.8 mol). Water (1280 g) and 2-methyltetrahydrofuran (547 g) were successively added and the mixture was cooled to 4 °C. 2-Methylbutanoyl chloride (373 g, 3.1 mol) was added dropwise over the period of 1 hour, keeping the temperature of the reaction mixture below 15 °C. Following the addition, the mixture was stirred for one hour before the temperature was raised to 45 °C and a mixture of solid 2-chloro-1-(p-tolyl)ethan-1-one (97%, 571 g, 3.3 mol) and tetrabutylammonium bromide (9.98 g, 0.03 mol) was added in portions over 15 minutes. The mixture was then heated to 76 °C ($T_j$: 95 °C) for one hour and 30 minutes, after which GC analysis of the mixture showed complete conversion of 1-(2-methylbutanoyl)piperidine-2-carboxylic acid to 2-oxo-2-(p-tolyl)ethyl 1-(2-methylbutanoyl)piperidine-2-carboxylate. The reaction mixture was then cooled to 60 °C, the stirring was stopped and the water layer was removed from the turbid light orange organic layer through the bottom valve. 2-methyltetrahydrofuran (1025 g) was added to the mixture remaining in the reactor. The solution was stirred (160 rpm) and heated to reflux (85 °C, $T_j$: 110 °C) and ammonium acetate (1462 g, 18.6 mol, 98%) was added portionswise while removing the water azeotropically. The reaction mixture was stirred at reflux overnight until analysis by GC showed full conversion and the water produced by the reaction was removed azeotropically. The reaction mixture was cooled to 60 °C and washed once with water (1000 g) and three times with sodium hydroxide (2M, 1000 g) and four times with water (1000 g) to reach pH 7. The product solution was heated to reflux again to azeotropically dry the solution (Tj: 115 °C) and then the solution was cooled to 0 °C (Tj: 0 °C) and stirring (50 rpm) was continued for 24 hours. The fine crystals that formed in the orange mixture were removed by filtration of the mass through a Büchner funnel and then washed twice with cold methyl-t-butylether (4 °C, 400 ml).

The solid product was dried in vacuum to give 2-methyl-1-(2-(5-(p-tolyl)-1H-imidazol-2-yl)piperidin-1-yl)butan-1-one (390 g, 1.2 mol, 39% yield) as a white solid. M.p.: 156.3 °C. GC/MS (EI): m/z (%): 325 (10) [M+], 268 (2), 240 (100), 224 (3), 185 (10), 159 (2), 142 (1), 84 (2), 57 (4). $^1$H NMR (500 MHz, DMSO-d6, 413K, mixture of stereoisomers and tautomers) δ = 11.32 (br s, 1H), 7.61(br m, 2H), 7.28 (br s, 1H), 7.14 (br m, 2H), 5.63 (br m, 1H), 4.06 (br m, 1H), 3.29 (br m, 1H), 2.76 (m, 1H), 2.31 (s, 3H), 2.23 (br m, 1H), 1.83 - 1.60 (m, 5H), 1.49 - 1.33 (m, 2H), 1.09 - 1.05 (2d, *J* = 6.8 Hz, 3H), 0.91 - 0.85 (2t, *J* = 7.4 Hz, 3H) ppm. $^{13}$C NMR (125 MHz, DMSO-d6, 413K, mixture of stereoisomers and tautomers, shifts extracted from HSQC & HMBC experiments) δ 174.4 (s), 146.8 (s), 139.6 (s), 134.3 (s), 132.1 (s),

128.1 (2d), 123.7 (2d), 110.9 (d), 47.5 ( d), 40.1 (t), 35.6 (d), 27.6 (t), 26.0 (t), 24.7 (t), 19.8 (q), 19.0 (t), 16.3 (q), 10.5 (q) ppm.

The foregoing broadly describes certain embodiments of the present invention without limitation. Variations and modifications as will be readily apparent to those skilled in the art are intended to be within the scope of the present invention as defined in and by the appended claims.

## Claims

1. A method for the *in-situ* formation of a compound of formula (V) from pipecolic acid, wherein the *in-situ* method takes place in the presence of a solvent, wherein the solvent is an organic solvent having a boiling point ranging from about 50°C to about 160°C, water, or a mixture thereof, wherein the method comprises:

   (a) reacting pipecolic acid with an acid chloride of formula (Ia) in the presence of a base, or reacting pipecolic acid with an acid anhydride of formula (Ib), optionally in the presence of a base, to form a compound of formula (II),

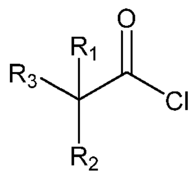

Formula (Ia)

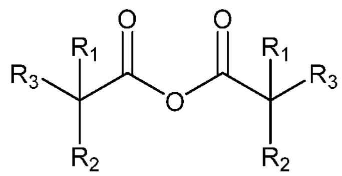

Formula (Ib)

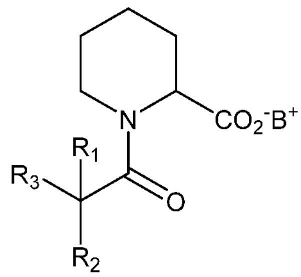

Formula (II)

   (b) reacting the compound of formula (II) with a compound of formula (III) to form a compound of formula (IV),

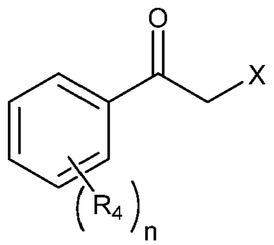

Formula (III)

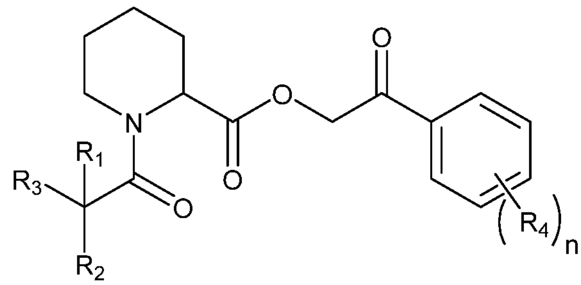

Formula (IV)

   (c) reacting the compound of formula (IV) with an ammonium source to form a compound of formula (V),

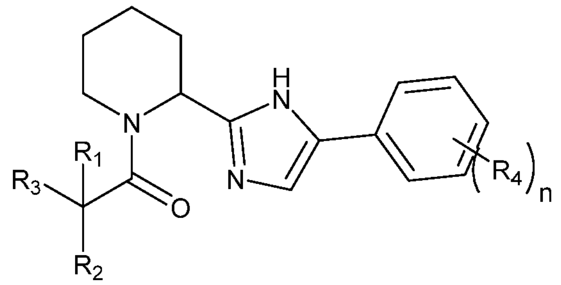

Formula (V)

   wherein

   $R_1$, $R_2$ and $R_3$ together with the carbon atom to which they are attached form a hydrocarbon group optionally

comprising up to 3 heteroatoms independently selected from O, S, N and F;
the phenyl group of the compounds of formulas (III), (IV) and (V) is substituted with n $R_4$ substituents wherein n is zero, one, two, three, four, or five;
each $R_4$ is independently selected from halogen, cyano, nitro, $C_1$-$C_6$ alkyl optionally comprising up to 5 halogen atoms, $C_2$-$C_6$ alkenyl, $C_1$-$C_6$ alkoxy optionally comprising up to 3 halogen atoms, $C_1$-$C_3$-alkoxy-$C_1$-$C_3$-alkyl, and $C_3$-$C_7$ cycloalkyl;
$B^+$ is a cation provided by the base; and
X is a halogen.

2. The method of claim 1, wherein the base is a metal phosphate, a metal hydroxide, a metal carbonate, metal bicarbonate, or a combination thereof.

3. The method of claim 1 or 2, wherein the base is sodium hydroxide or potassium hydroxide.

4. The method of any preceding claim, wherein at least two equivalents of base to the acid chloride of formula (Ia) or acid anhydride of formula (Ib) or compound of formula (III) are used.

5. The method of any preceding claim, wherein the reaction of the pipecolic acid with the acid chloride of formula (Ia) or acid anhydride of formula (Ib) to form the compound of formula (II), and/or the reaction of the compound of formula (II) with the compound of formula (III) to form the compound of formula (IV), takes place in the presence of a phase transfer catalyst.

6. The method of claim 5, wherein the phase transfer catalyst is tetrabutylammonium bromide (TBAB).

7. The method of any preceding claim, wherein the solvent is an aromatic solvent, for example an alkylbenzene solvent such as toluene.

8. The method of any one of claim 1 to 6, wherein the solvent is a non-aromatic solvent solvent, for example 2-methyltetrahydrofuran.

9. The method of any preceding claim, wherein the reaction of the compound of formula (II) with the compound of formula (III) to form the compound of formula (IV) takes place at a temperature ranging from about 50°C to about 120°C.

10. The method of any preceding claim, wherein the ammonium source is ammonium acetate or a mixture of ammonia and acetic acid.

11. The method of any preceding claim, wherein the reaction of the compound of formula (IV) with the ammonium source to form a compound of formula (V) takes place at a temperature ranging from about 90°C to about 120°C.

12. The method of any preceding claim, wherein the carbon atom to which $R_1$, $R_2$ and $R_3$ are attached is a chiral center and the chirality remains unchanged throughout the reaction.

13. The method of any preceding claim, wherein:

$R_1$ is selected from hydrogen and methyl; and/or
$R_2$ is selected from hydrogen, $C_1$-$C_2$ alkyl and $C_2$-$C_3$ alkenyl; and/or
$R_3$ is selected from $C_1$-$C_3$ alkyl, $C_2$-$C_5$ alkenyl containing one or two double bonds, $C_1$-$C_3$ alkoxy, $C_1$-$C_4$ alkyl-C(O)-, $C_1$-$C_4$ alkyl-S-, $C_1$-$C_4$ alkyl-$SCH_2$-, $C_1$-$C_4$ alkenyl-S-, $C_1$-$C_4$ alkyl-S(O)-, $C_1$-$C_4$ alkyl-$S(O)_2$-, $C_1$-$C_4$ alkenyl-S(O)-, $C_1$-$C_4$ alkenyl-$S(O)_2$-, -SH, $CF_3S$-, cyclopropyl, cyclobutyl, furyl optionally substituted with methyl, and $C_1$-$C_6$ fluoro-alkyl.

14. The method of any preceding claim, wherein $R_1$, $R_2$ and $R_3$ together with the carbon atom to which they are attached form a hydrocarbon group selected from 3-thiabut-2-yl, 2-methyl-3-thiabut-2-yl, 3-thiapent-2-yl, 4-thiapent-2-yl, 2-thiaprop-1-yl, 2-methyl-3-thiapent-2-yl, 3-oxo-3-thiabut-2-yl, 3-oxo-2-methyl-3-thiabut-2-yl, 3-oxo-3-thiapent-2-yl, 4-oxo-4-thiapent-2-yl, 2-oxo-2-thiaprop-1-yl, 3-oxo-2-methyl-3-thiapent-2-yl, but-2-yl, pent-2-yl, but-3-en-2-yl, pent-3-en-2-yl, but-2-en-2-yl, pent-2-en-2-yl, but-1-en-2-yl, pent-1-en-2-yl, 2-methylbut-2-yl, 2-methylpent-2-yl, 2-methylbut-3-en-2-yl, 3-methylbut-2-yl, 3-methylbut-3-en-2-yl, 3-methylbut-2-en-2-yl, 2,3-dimethylbut-2-yl, 2,3-dimethylpent-2-yl, 2,3-dimethylbut-3-en-2-yl, 2,3-dimethylpent-3-en-2-yl, 2-methylpent-3-en-2-yl, prop-2-yl, prop-1-yl, ethyl, cyclopropyl, 1,1-dimethylcycloprop-2-yl, 1-methylcycloprop-2-yl, 1-methylcycloprop-1-yl, 3-thiahex-5-en-2-yl, 2-methyl-3-thiahex-5-en-2-yl, 1-mercaptoeth-1-yl, 2-mercaptoprop-2-yl, 3,3,3-trifluoroprop-2-yl, 2-methyl-3,3,3-trifluoroprop-2-yl, 1-(2-furyl)eth-1-yl, 1-(5-methylfur-2-yl)eth-1-yl, 2-(2-furyl)prop-2-yl, 1-(3-furyl)eth-1-yl, 1-(5-methylfur-3-yl)eth-1-yl, 2-(3-furyl)prop-2-yl, 1-(2-tetrahydrofuryl)eth-1-yl, 2-(2-tetrahydrofuryl)prop-2-yl, 1-(3-tetrahydrofuryl)eth-1-yl, 2-(3-tetrahydrofuryl)prop-2-yl, 1-cyclopropyleth-1-yl, 2-cyclopropylprop-2-yl, 1-cyclobutyleth-1-yl, 2-cyclobutylprop-2-yl, cyclobutyl, cyclopentyl, pent-2-en-3-yl, 1-methoxyprop-1-yl, 1-methoxyeth-1-yl, 1,1,1-trifluorobut-3-yl, and 3-thiacyclobut-1-yl.

**15.** The method of any preceding claim, wherein the compound of formula (V) is 2-methyl-1-(2-(5-(p-tolyl)-1H-imidazol-2-yl)piperidin-1-yl)butan-1-one, 2-(methylthio)-1-(2-(5-(p-tolyl)-1H-imidazol-2-yl)piperidin-1-yl)propan-1-one, 2-methyl-2-(methylthio)-1-(2-(5-(p-tolyl)-1H-imidazol-2-yl)piperidin-1-yl)propan-1-one, or 2,2-dimethyl-1-(2-(5-(p-tolyl)-1H-imidazol-2-yl)piperidin-1-yl)but-3-en-1-one.

## Patentansprüche

**1.** Verfahren zur *in situ*-Bildung einer Verbindung der Formel (V) aus Pipecolinsäure, wobei das *in situ*-Verfahren in Gegenwart eines Lösungsmittels stattfindet, wobei das Lösungsmittel ein organisches Lösungsmittel mit einem Siedepunkt im Bereich von etwa 50 °C bis etwa 160 °C, Wasser oder eine Mischung davon ist, wobei das Verfahren umfasst:

(a) Umsetzen von Pipecolinsäure mit einem Säurechlorid der Formel (Ia) in Gegenwart einer Base, oder Umsetzen von Pipecolinsäure mit einem Säureanhydrid der Formel (Ib), gegebenenfalls in Gegenwart einer Base, um eine Verbindung der Formel (II) zu bilden:

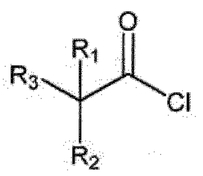

Formel (Ia)

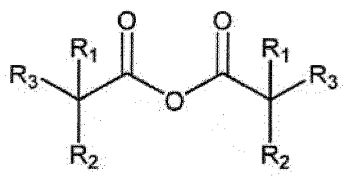

Formel (Ib)

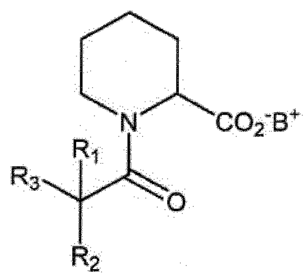

Formel (II)

(b) Umsetzen der Verbindung der Formel (II) mit einer Verbindung der Formel (III), um eine Verbindung der Formel (IV) zu bilden:

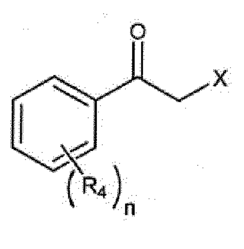

Formel (III)

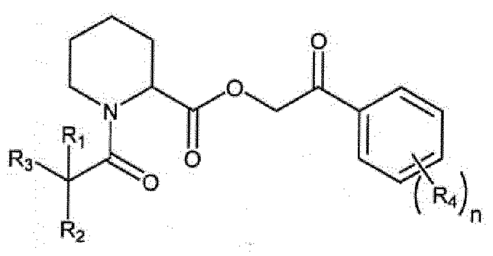

Formel (IV)

(c) Umsetzen der Verbindung der Formel (IV) mit einer Ammoniakquelle, um eine Verbindung der Formel (V) zu bilden:

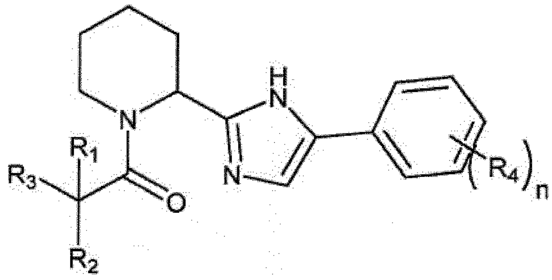

Formel (V)

wobei

$R_1$, $R_2$ und $R_3$ zusammen mit dem Kohlenstoffatom, an das sie gebunden sind, eine Kohlenwasserstoffgruppe bilden, die gegebenenfalls bis zu 3 Heteroatome umfasst, die unabhängig ausgewählt sind aus O, S, N und F;
die Phenylgruppe der Verbindungen der Formeln (III), (IV) und (V) mit n Substituenten $R_4$ substituiert ist, wobei n Null, eins, zwei, drei, vier oder fünf ist;
jedes $R_4$ unabhängig ausgewählt ist aus Halogen, Cyano, Nitro, $C_1$-$C_6$-Alkyl, gegebenenfalls umfassend bis zu 5 Halogenatome, $C_2$-$C_6$-Alkenyl, $C_1$-$C_6$-Alkoxy, gegebenenfalls umfassend bis zu 3 Halogenatome, $C_1$-$C_3$-Alkoxy-$C_1$-$C_3$-alkyl und $C_3$-$C_7$-Cycloalkyl;
$B^+$ ein Kation ist, das durch die Base bereitgestellt wird; und
X ein Halogen ist.

**2.** Verfahren nach Anspruch 1, wobei die Base ein Metallphosphat, ein Metallhydroxid, ein Metallcarbonat, Metallbicarbonat oder eine Kombination davon ist.

**3.** Verfahren nach Anspruch 1 oder 2, wobei die Base Natriumhydroxid oder Kaliumhydroxid ist.

**4.** Verfahren nach einem der vorhergehenden Ansprüche, wobei mindestens zwei Äquivalente Base zu dem Säurechlorid der Formel (Ia) oder Säureanhydrid der Formel (Ib) oder der Verbindung der Formel (III) verwendet werden.

**5.** Verfahren nach einem der vorhergehenden Ansprüche, wobei die Umsetzung der Pipecolinsäure mit dem Säurechlorid der Formel (Ia) oder Säureanhydrid der Formel (Ib), um die Verbindung der Formel (II) zu bilden, und/oder die Umsetzung der Verbindung der Formel (II) mit der Verbindung der Formel (III), um die Verbindung der Formel (IV) zu bilden, in Gegenwart eines Phasentransferkatalysators stattfindet.

**6.** Verfahren nach Anspruch 5, wobei der Phasentransferkatalysator Tetrabutylammoniumbromid (TBAB) ist.

**7.** Verfahren nach einem der vorhergehenden Ansprüche, wobei das Lösungsmittel ein aromatisches Lösungsmittel ist, beispielsweise ein Alkylbenzollösungsmittel, wie Toluol.

**8.** Verfahren nach einem der Ansprüche 1 bis 6, wobei das Lösungsmittel ein nicht-aromatisches Lösungsmittel ist, beispielsweise 2-Methyltetrahydrofuran.

**9.** Verfahren nach einem der vorhergehenden Ansprüche, wobei die Umsetzung der Verbindung der Formel (II) mit der Verbindung der Formel (III), um die Verbindung der Formel (IV) zu bilden, bei einer Temperatur im Bereich von etwa 50 °C bis etwa 120 °C stattfindet.

**10.** Verfahren nach einem der vorhergehenden Ansprüche, wobei die Ammoniumquelle Ammoniumacetat oder eine Mischung aus Ammoniak und Essigsäure ist.

**11.** Verfahren nach einem der vorhergehenden Ansprüche, wobei die Umsetzung der Verbindung der Formel (IV) mit der Ammoniumquelle, um eine Verbindung der Formel (V) zu bilden, bei einer Temperatur im Bereich von etwa 90 °C bis etwa 120 °C stattfindet.

**12.** Verfahren nach einem der vorhergehenden Ansprüche, wobei das Kohlenstoffatom, an das $R_1$, $R_2$ und $R_3$ gebunden

sind, ein chirales Zentrum ist, und die Chiralität während der Umsetzung unverändert bleibt.

**13.** Verfahren nach einem der vorhergehenden Ansprüche, wobei:

$R_1$ ausgewählt ist aus Wasserstoff und Methyl; und/oder $R_2$ ausgewählt ist aus der Gruppe bestehend aus Wasserstoff, $C_1$-$C_2$-Alkyl- und $C_2$-$C_3$-Alkenyl; und/oder
$R_3$ ausgewählt ist aus $C_1$-$C_3$-Alkyl, $C_2$-$C_5$-Alkenyl, das eine oder zwei Doppelbindungen enthält, $C_1$-$C_3$-Alkoxy, $C_1$-$C_4$-Alkyl-C(O)-, $C_1$-$C_4$-Alkyl-S-, $C_1$-$C_4$-Alkyl-$SCH_2$-, $C_1$-$C_4$-Alkenyl-S-, $C_1$-$C_4$-Alkyl-S(O)-, $C_1$-$C_4$-Alkyl-$S(O)_2$-, $C_1$-$C_4$-Alkenyl-S(O)-, $C_1$-$C_4$-Alkenyl-$S(O)_2$-, -SH, $CF_3S$-, Cyclopropyl, Cyclobutyl, Furyl, gegebenenfalls substituiert mit Methyl, und $C_1$-$C_6$-Fluoralkyl.

**14.** Verfahren nach einem der vorhergehenden Ansprüche, wobei $R_1$, $R_2$ und $R_3$ zusammen mit dem Kohlenstoffatom, an das sie gebunden sind, eine Kohlenwasserstoffgruppe bilden, die ausgewählt ist aus 3-Thiabut-2-yl, 2-Methyl-3-thiabut-2-yl, 3-Thiapent-2-yl, 4-Thiapent-2-yl, 2-Thiaprop-1-yl, 2-Methyl-3-thiapent-2-yl, 3-Oxo-3-thiabut-2-yl, 3-Oxo-2-methyl-3-thiabut-2-yl, 3-Oxo-3-thiapent-2-yl, 4-Oxo-4-thiapent-2-yl, 2-Oxo-2-thiaprop-1-yl, 3-Oxo-2-methyl-3-thiapent-2-yl, But-2-yl, Pent-2-yl, But-3-en-2-yl, Pent-3-en-2-yl, But-2-en-2-yl, Pent-2-en-2-yl, But-1-en-2-yl, Pent-1-en-2-yl, 2-Methylbut-2-yl, 2-Methylpent-2-yl, 2-Methylbut-3-en-2-yl, 3-Methylbut-2-yl, 3-Methylbut-3-en-2-yl, 3-Methylbut-2-en-2-yl, 2,3-Dimethylbut-2-yl, 2,3-Dimethylpent-2-yl, 2,3-Dimethylbut-3-en-2-yl, 2,3-Dimethylpent-3-en-2-yl, 2-Methylpent-3-en-2-yl, Prop-2-yl, Prop-1-yl, Ethyl, Cyclopropyl, 1,1-Dimethylcycloprop-2-yl, 1-Methylcycloprop-2-yl, 1- Methylcycloprop-1-yl, 3-Thiahex-5-en-2-yl, 2-Methyl-3-thiahex-5-en-2-yl, 1-Mercaptoeth-1-yl, 2-Mercaptoprop-2-yl, 3,3,3-Trifluorprop-2-yl, 2-Methyl-3,3,3-trifluorprop-2-yl, 1-(2-Furyl)eth-1-yl, 1-(5-Methylfur-2-yl)eth-1-yl, 2-(2-Furyl)prop-2-yl, 1-(3-Furyl)eth-1-yl, 1-(5-Methylfur-3-yl)eth-1-yl, 2-(3-Furyl)prop-2-y1, 1-(2-Tetrahydrofuryl)eth-1-yl, 2-(2-Tetrahydrofuryl)prop-2-yl, 1-(3-Tetrahydrofuryl)eth-1-yl, 2-(3-Tetrahydrofuryl)prop-2-yl, 1-Cyclopropyleth-1-yl, 2-Cyclopropylprop-2-yl, 1-Cyclobutyleth-1-yl, 2-Cyclobutylprop-2-yl, Cyclobutyl, Cyclopentyl, Pent-2-en-3-yl, 1-Methoxyprop-1-yl, 1-Methoxyeth-1-yl, 1,1,1-Trifluorbut-3-yl und 3-Thiacyclobut-1-yl.

**15.** Verfahren nach einem der vorhergehenden Ansprüche, wobei die Verbindung der Formel (V) 2-Methyl-1-(2-(5-(p-tolyl)-1H-imidazol-2-yl)piperidin-1-yl)butan-1-on, 2-(Methylthio)-1-(2-(5-(p-tolyl)-1H-imidazol-2-yl)piperidin-1-yl)propan-1-on, 2-Methyl-2-(methylthio)-1-(2-(5-(p-tolyl)-1H-imidazol-2-yl)piperidin-1-yl)propan-1-on oder 2,2-Dimethyl-1-(2-(5-(p-tolyl)-1H-imidazol-2-yl)piperidin-1-yl)but-3-en-1-on ist.

## Revendications

**1.** Procédé pour la formation *in situ* d'un composé de formule (V) à partir de l'acide pipécolique, le procédé *in situ* ayant lieu en la présence d'un solvant, le solvant étant un solvant organique ayant un point d'ébullition dans la plage d'environ 50 °C à environ 160 °C, l'eau, ou un mélange correspondant, le procédé comprenant :

(a) la mise en réaction d'acide pipécolique avec un chlorure d'acide de formule (Ia) en la présence d'une base, ou la mise en réaction d'acide pipécolique avec un anhydride d'acide de formule (Ib), éventuellement en la présence d'une base, pour former un composé de formule (II),

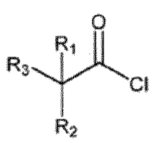
formule (Ia)

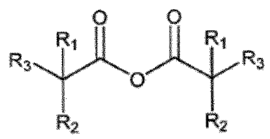
formule (Ib)

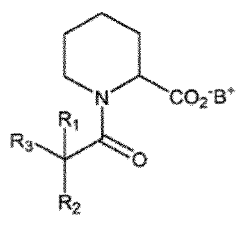
formule (II)

(b) la mise en réaction du composé de formule (II) avec un composé de formule (III) pour former un composé de formule (IV),

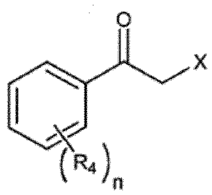

formule (III)

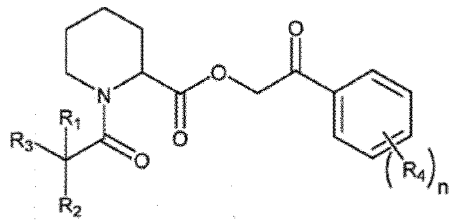

formule (IV)

(c) la mise en réaction du composé de formule (IV) avec une source d'ammonium pour former un composé de formule (V)

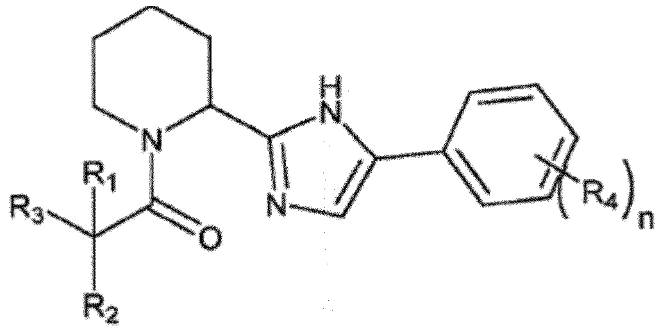

formule (V)

$R_1$, $R_2$ et $R_3$, conjointement avec l'atome de carbone auquel ils sont fixés, formant un groupe hydrocarboné comprenant éventuellement jusqu'à 3 hétéroatomes indépendamment choisis parmi O, S, N et F ;
le groupe phényle des composés des formules (III), (IV) et (V) étant substitué par n substituants $R_4$, n étant zéro, un, deux, trois, quatre ou cinq ;
chaque $R_4$ étant indépendamment choisi parmi halogène, cyano, nitro, Ci-Ce alkyle comprenant éventuellement jusqu'à 5 atomes d'halogène, $C_2$-$C_6$ alcényle, Ci-Ce alcoxy éventuellement comprenant jusqu'à 3 atomes d'halogène, $C_1$-$C_3$-alcoxy-$C_1$-$C_3$-alkyle, et $C_3$-$C_7$ cycloalkyle ;
$B^+$ étant un cation fourni par la base; et
X étant un halogène.

**2.** Procédé selon la revendication 1, la base étant un phosphate métallique, un hydroxyle métallique, un carbonate métallique, un bicarbonate métallique ou une combinaison correspondante.

**3.** Procédé selon la revendication 1 ou 2, la base étant l'hydroxyde de sodium ou l'hydroxyde de potassium.

**4.** Procédé selon une quelconque revendication précédente, au moins deux équivalents de base par rapport au chlorure d'acide de formule (Ia) ou à l'anhydride d'acide de formule (Ib) ou le composé de formule (III) étant utilisés.

**5.** Procédé selon une quelconque revendication précédente, la réaction de l'acide pipécolique avec le chlorure d'acide de formule (Ia) ou l'anhydride d'acide de formule (Ib) pour former le composé de formule (II), et/ou la réaction du composé de formule (II) avec le composé de formule (III) pour former le composé de formule (IV), ayant lieu en la présence d'un catalyseur de transfert de phase.

**6.** Procédé selon la revendication 5, le catalyseur de transfert de phase étant le bromure de tétrabutylammonium (TBAB) .

**7.** Procédé selon une quelconque revendication précédente, le solvant étant un solvant aromatique, par exemple un solvant de type alkylbenzène tel que le toluène.

**8.** Procédé selon l'une quelconque des revendications 1 à 6, le solvant étant un solvant de type solvant non aromatique, par exemple le 2-méthyltétrahydrofuranne.

**9.** Procédé selon une quelconque revendication précédente, la réaction du composé de formule (II) avec le composé de formule (III) pour former le composé de formule (IV) ayant lieu à une température dans la plage d'environ 50 °C à environ 120 °C.

**10.** Procédé selon une quelconque revendication précédente, la source d'ammonium étant l'acétate d'ammonium ou un mélange d'ammoniac et d'acide acétique.

**11.** Procédé selon une quelconque revendication précédente, la réaction du composé de formule (IV) avec la source d'ammonium pour former un composé de formule (V) ayant lieu à une température dans la plage d'environ 90 °C à environ 120 °C.

**12.** Procédé selon une quelconque revendication précédente, l'atome de carbone auquel $R_1$, $R_2$ et $R_3$ sont fixés étant un centre chiral et la chiralité restant inchangée pendant toute la réaction.

**13.** Procédé selon une quelconque revendication précédente,

$R_1$ étant choisi parmi hydrogène et méthyle ; et/ou
$R_2$ étant choisi parmi hydrogène, $C_1$-$C_2$ alkyle et $C_2$-$C_3$ alcényle ; et/ou
$R_3$ étant choisi parmi $C_1$-$C_3$ alkyle, $C_2$-$C_5$ alcényle contenant une ou deux doubles liaisons, $C_1$-$C_3$ alcoxy, $C_1$-$C_4$ alkyl-C(O)-, $C_1$-$C_4$ alkyl-S-, $C_1$-$C_4$ alkyl-$SCH_2$-, $C_1$-$C_4$ alcényl-S-, $C_1$-$C_4$ alkyl-S(O)-, $C_1$-$C_4$ alkyl-$S(O)_2$-, $C_1$-$C_4$ alcényl-S(O)-, $C_1$-$C_4$ alcényl-$S(O)_2$-, -SH, $CF_3S$-, cyclopropyle, cyclobutyle, furyle éventuellement substitué par méthyle, et Ci-Ce fluoro-alkyle.

**14.** Procédé selon une quelconque revendication précédente, $R_1$, $R_2$ et $R_3$, conjointement avec l'atome de carbone auquel ils sont fixés, formant un groupe hydrocarboné choisi parmi 3-thiabut-2-yle, 2-méthyl-3-thiabut-2-yle, 3-thiapent-2-yle, 4-thiapent-2-yle, 2-thiaprop-1-yle, 2-méthyl-3-thiapent-2-yle, 3-oxo-3-thiabut-2-yle, 3-oxo-2-méthyl-3-thiabut-2-yle, 3-oxo-3-thiapent-2-yle, 4-oxo-4-thiapent-2-yle, 2-oxo-2-thiaprop-1-yle, 3-oxo-2-méthyl-3-thiapent-2-yle, but-2-yle, pent-2-yle, but-3-én-2-yle, pent-3-én-2-yle, but-2-én-2-yle, pent-2-én-2-yle, but-1-én-2-yle, pent-1-én-2-yle, 2-méthylbut-2-yle, 2-méthylpent-2-yle, 2-méthylbut-3-én-2-yle, 3-méthylbut-2-yle, 3-méthylbut-3-én-2-yle, 3-méthylbut-2-én-2-yle, 2,3-diméthylbut-2-yle, 2,3-diméthylpent-2-yle, 2,3-diméthylbut-3-én-2-yle, 2,3-diméthylpent-3-én-2-yle, 2-méthylpent-3-én-2-yle, prop-2-yle, prop-1-yle, éthyle, cyclopropyle, 1,1-diméthylcycloprop-2-yle, 1-méthylcycloprop-2-yle, 1-méthylcycloprop-1-yle, 3-thiahex-5-én-2-yle, 2-méthyl-3-thiahex-5-én-2-yle, 1-mercaptoéth-1-yle, 2-mercaptoprop-2-yle, 3,3,3-trifluoroprop-2-yle, 2-méthyl-3,3,3-trifluoroprop-2-yle, 1-(2-furyl)éth-1-yle, 1-(5-méthylfur-2-yl)éth-1-yle, 2-(2-furyl)prop-2-yle, 1-(3-furyl)éth-1-yle, 1-(5-méthylfur-3-yl)éth-1-yle, 2-(3-furyl)prop-2-yle, 1-(2-tétrahydrofuryl)éth-1-yle, 2-(2-tétrahydrofuryl)prop-2-yle, 1-(3-tétrahydrofuryl)éth-1-yle, 2-(3-tétrahydrofuryl)prop-2-yle, 1-cyclopropyléth-1-yle, 2-cyclopropylprop-2-yle, 1-cyclobutyléth-1-yle, 2-cyclobutylprop-2-yle, cyclobutyle, cyclopentyle, pent-2-én-3-yle, 1-méthoxyprop-1-yle, 1-méthoxyéth-1-yle, 1,1,1-trifluorobut-3-yle et 3-thiacyclobut-1-yle.

**15.** Procédé selon une quelconque revendication précédente, le composé de formule (V) étant 2-méthyl-1-(2-(5-(p-tolyl)-1H-imidazol-2-yl)pipéridin-1-yl)butan-1-one, 2-(méthylthio)-1-(2-(5-(p-tolyl)-1H-imidazol-2-yl)pipéridin-1-yl)propan-1-one, 2-méthyl-2-(méthylthio)-1-(2-(5-(p-tolyl)-1H-imidazol-2-yl)pipéridin-1-yl)propan-1-one, ou 2,2-diméthyl-1-(2-(5-(p-tolyl)-1H-imidazol-2-yl)pipéridin-1-yl)but-3-én-1-one.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015047973 A